# EUROPEAN PATENT SPECIFICATION

(11) **EP 4 743 102 B1**
(45) Date of publication and mention of the grant of the patent: **21.10.2026**
(21) Application number: 25719776.4
(22) Date of filing: 14.04.2025
(51) Int. Cl.: A61K 38/05, A61K 38/06, A61P 25/28, A61P 43/00

(54) **COLLAGEN PEPTIDE COMPOSITION AND USE THEREOF IN AMELIORATING NEUROLOGICAL CONDITIONS AND FIBROSIS**
KOLLAGENPEPTIDZUSAMMENSETZUNG UND VERWENDUNG DAVON ZUR LINDERUNG VON NEUROLOGISCHEN ERKRANKUNGEN UND FIBROSE
COMPOSITION PEPTIDIQUE DE COLLAGÈNE ET SON UTILISATION POUR FAIRE RÉGRESSER DES PATHOLOGIES NEUROLOGIQUES ET LA FIBROSE

(30) Priority: 15.04.2024 BE 202405214; 15.04.2024 BE 202405215
(43) Date of publication of application: 20.05.2026
(73) Proprietor: Rousselot BV, 9000 Gent (BE)
(72) Inventor: VLEMINCKX, Sara, 9000 GENT (BE); GEVAERT, Elien Monique, 9000 GENT (BE); VERGAUWEN, Bjorn, 9000 GENT (BE)
(74) Representative: EP&C
(86) International application number: PCT/EP2025/060295
(87) International publication number: WO 2025/219352

(56) References cited:
- WO-A1-2021/100614
- WO-A2-2018/094023
- JP-A- 2014 125 427
- JP-A- 2022 177 701
- JP-B1- 6 598 412
- FREISE CHRISTIAN ET AL: "The synthetic hydroxyproline-containing collagen analogue (Gly-Pro-Hyp) 10 promotes enzymatic activity of matrixmetalloproteinase-2 in vitro", EUROPEAN JOURNAL OF MICROBIOLOGY AND IMMUNOLOGY, vol. 2, no. 3, 1 September 2012 (2012-09-01), HU, pages 186 - 191, XP093219306, ISSN: 2062-509X, DOI: 10.1556/EuJMI.2.2012.3.3

## Description

### TECHNICAL FIELD

The invention pertains to a collagen peptide composition enriched in cyclic(glycine-proline) and a method for obtaining the collagen peptide composition. The peptide composition can be used as active ingredient in food supplements.

### BACKGROUND OF THE INVENTION

Oxidative stress is increasingly considered a major mechanism that underlies the aetiology of a variety of conditions including brain-related conditions (e.g. neurological conditions, reduced cognitive ability) or pathological wound healing such as fibrosis (Shroff et al. Curr Pathobiol Rep. 2014; 2(4): 257-267; Antar et al. Int J Mol Sci. 2023 Feb 16;24(4):4004).

Cyclic(glycine-proline) (i.e. "cGP") is an endogenous diketopiperazine and cyclic dipeptide compound that may be derived from cyclization of glycine-proline-glutamate (naturally cleaved from the IGF-1 N-terminal). It is considered that cGP may exhibit anti-inflammatory effects and immune-modulatory effects, and may be capable of reducing oxidative stress and therefore suitable for ameliorating brain-related conditions or pathological wound healing.

As a drawback, the synthesis of cyclic peptides such as cGP can be complex and expensive. Conventional methods to produce cGP includes their synthesis from amino acids followed by cyclization. These conventional methods are typically complicated because of the need to remove the organic solvent and resin used for solid-phase synthesis. The cost of production may be a limiting factor in the development and accessibility of cGP for therapeutic use.

As another drawback, isolating the CTP is considered to remove most of the dipeptides and free amino acids, which typically contribute to the biological effect of collagen peptide compositions. The beneficial effects of cGP, when used alone, appear insufficient (compared to e.g. pharmacological interventions) to lead to its widespread use. Thus, there is a need for a safe and more effective active ingredients or compositions.

The object for the present invention is to provide a novel active ingredient or composition which can overcome one or more of aforementioned limitations, and which is preferably obtainable by natural materials and/or which can be provided as a functional food or food supplement. It is a particular objection of the present invention to provide a safe and effective active ingredient or composition suitable for ameliorating brain-related conditions (e.g. neurological conditions, reduced cognitive ability) or pathological wound healing such as fibrosis.

### SUMMARY OF THE INVENTION

The present inventors discovered a peptide composition obtainable from collagen and which exerts strong anti-oxidative, anti-(neuro)inflammatory and anti-fibrotic properties. The peptide composition is found to be effective, among others, as active ingredient for ameliorating neurological diseases, in improving/preventing decline in normal cognitive functions such as ability in learning and memory, and/or preventing and/or treating fibrosis.

The peptide composition of the invention is characterized by its particularly high concentration of cyclic(glycine-proline) (i.e. cGP) and further content of (cyclic) di- and tripeptides, in particular the presence of glycine-proline hydroxyproline and glycine-proline-alanine tripeptide (in their relative amounts).

The present inventors found that heating of an intermediate collagen hydrolysate rich in glycine-proline-x tripeptide sequences - in particular rich in glycine-proline hydroxyproline (e.g. 1.0x10⁴ - 1.0x10⁵ ppm) and rich in glycine-proline-alanine tripeptide (e.g. 5x10³ - 5x10⁴ ppm) sequences - achieves a peptide composition enriched in ~20 times more cGP (e.g. 1.0x10⁴ - 1.0x10⁵ ppm) and further comprising desirable amounts of (cyclic) di- and tripeptides.

Present inventors furthermore found that cyclisation by heating led to changes in relatively lower molecular weight fractions, which in particular resulted in a peptide composition with a characteristic peak in the 250-450 Da molecular weight range which was not apparent before cyclisation. It is considered that cGP (Mw=154 Da) contributes to this characteristic peak because cGP, due to its shape, shows a typical peak in a size exclusion chromatogram at 250-450 Da. Thus, the molecular weight distributions before and after cyclisation are also characteristic for the products disclosed herein.

The cGP and the further peptides in the peptide composition were surprisingly found to have synergistic anti-oxidative, anti-(neuro)inflammatory properties and anti-fibrotic properties. In particular, synergistic anti-oxidative and anti-(neuro)inflammatory and anti-fibrotic effects were discovered for the combination of cGP, glycine-proline-hydroxyproline and glycine-proline-alanine tripeptides. Without being bound by theory, the present inventors consider that further collagen dipeptides may also contribute to the anti-oxidative properties. In addition or alternatively, the combination of the various peptides may together provide highest health benefits.

In a first aspect, the present invention pertains to a peptide composition, optionally obtainable by the method disclosed herein, the composition comprising 1.0x10⁴ ppm or more cyclic(glycine-proline), preferably 1.0x10⁴ ppm - 1.0x10⁵ ppm cyclic(glycine-proline), 1.0x10³ ppm or more glycine-proline-hydroxyproline tripeptide, preferably 1.0x10³ - 1.0x10⁴ ppm glycine-proline-hydroxyproline tripeptide, and/or 1.0x10¹ ppm or more glycine-proline-alanine tripeptide, preferably 1.0x10¹ - 1.0x10³ ppm glycine-proline-alanine tripeptide, wherein the ppm is calculated on the total weight of peptides in the peptide composition.

In a second aspect, the present invention pertains to a method for obtaining a peptide composition comprising cyclic(glycine-proline), the method comprising
a) providing a collagen hydrolysate comprising 1.0x10⁴ ppm -1.0x10⁵ ppm glycine-proline-hydroxyproline tripeptide and 5x10³ -5x10⁴ ppm glycine-proline-alanine, calculated on total weight of peptides in the collagen hydrolysate and having a weight-average molecular weight of at least 500 Da and at most 3000 Da; and
b) heat-treating the collagen hydrolysate at a temperature of at least 65 °C to obtain a peptide composition enriched in cyclic(glycine-proline).

In a third aspect, the present invention pertains to the peptide composition optionally obtainable by the method disclosed herein, for use as a medicament.

In a fourth aspect, the present invention pertains to the peptide composition optionally obtainable by the method disclosed herein, for use in preventing and/or treating a condition selected from the group consisting of a neurological condition and fibrosis.

In a fifth aspect, the present invention pertains to the non-therapeutic use of the peptide composition disclosed herein, in non-therapeutic improvement of cognitive function.

Described is a topical formulation comprising the peptide composition disclosed herein.

Described is a cosmetic composition comprising the peptide composition disclosed herein, e.g. suitable for non-therapeutic improvement of skin appearance in a fibrotic condition.

Described is a substrate coated with the peptide composition defined herein, wherein the substrate is preferably a medical implant.

### DETAILLED DESCRIPTION OF THE INVENTION

### Peptide composition

Various embodiments of the invention are exemplified by a peptide composition optionally obtainable by the method as disclosed herein.

When referring to a peptide composition, the ppm in the present disclosure is calculated on total weight of peptides in the peptide composition, unless indicated otherwise. When referring to a specific collagen hydrolysate, the ppm in the present disclosure is calculated on total weight of peptides in the collagen hydrolysate, unless indicated otherwise.

In an embodiment, the peptide composition of the present invention comprises:
- cyclic(glycine-proline): 1.0x10⁴ ppm or more;
- glycine-proline-hydroxyproline tripeptide: 1.0x10³ ppm or more; and/or
- glycine-proline-alanine tripeptide: 1.0x10¹ ppm or more.

In an embodiment, the peptide composition preferably comprises 1.0x10⁴ - 2.0x10⁵ ppm, such as 1.0x10⁴ - 1.0x10⁵ ppm, cyclic(glycine-proline).

In an embodiment, the peptide composition of the present invention comprises at least 1.0x10⁴ ppm cyclic(glycine-proline). Preferably, the peptide composition comprises 1.0x10⁴ - 1.0x10⁵ ppm cyclic(glycine-proline).

It is preferred in the context of the present invention to determine the concentration of a peptide in a composition by UPLC-MS analysis (ultra-performance liquid chromatography-mass spectrometry).

The term "cyclic(glycine-proline)" (i.e. cGP) in the context of the current invention means a cyclic dipeptide comprising the amino acids glycine and proline, typically formed by the condensation of a glycine- and (hydroxy)proline-containing linear dipeptide (e.g. Gly-Pro, Pro-Gly, Gly-Hyp or Hyp-Gly) or a linear tripeptide (e.g. Gly-Pro-Z, Pro-Gly-Z, Gly-Hyp-Z or Hyp-Gly-Z, wherein Z can be any amino acid. In cGP, the amino acids are linked together in a circular or closed-loop arrangement, typically through a peptide bond between the carboxyl group of glycine and the amino group of proline. In the context of the current invention, "cyclic glycine-proline" means the same and can be used interchangeably with "Cyclo(Gly-Pro)", "Cyclo(prolylglycyl)", "cyclic proline-glycine", "Cyclo (Pro-Gly)" and"Cyclo(glycylprolyl)".

In an embodiment, the peptide composition of the present invention comprises at least 1.0x10³ ppm glycine-proline-hydroxyproline tripeptide. Preferably, the peptide composition comprises 1.0x10³ - 1.0x10⁴ ppm glycine-proline-hydroxyproline tripeptide.

In an embodiment, the peptide composition of the present comprises at least 1.0x10¹ ppm glycine-proline-alanine tripeptide. Preferably, the peptide composition comprises 1.0x10¹ - 1.0x10³ ppm glycine-proline-alanine tripeptide.

1.0x10¹ as used herein means the same as 1.0x10 (i.e. 10). 1.0x10² as used herein means the same as 1.0x100 (i.e. 100). 1.0x10³ as used herein means the same as 1.0x1000 (i.e. 1000). 1.0x10⁴ as used herein means the same as 1.0x10000 (i.e. 10000). 1.0x10⁵ as used herein means the same as 1.0x100000 (i.e. 100000). 1.0x10⁸ as used herein means the same as 1.0x1000000 (i.e. 1000000).

In an embodiment, the peptide composition has a weight-average molecular weight of 800 - 2500 Da (Dalton). In an embodiment, the peptide composition has a weight-average molecular weight of 1000 - 2000 Da. In an embodiment, the peptide composition has a weight-average molecular weight of 1200-1800 Da, preferably 1500-1700 Da.

The peptide composition preferably has a weight-average molecular weight (Mw) in the range of 500 Da - 5000 Da, preferably 750 Da - 3000 Da, more preferably 1000 - 2500 Da, even more preferably 1200 - 2000 Da. In addition or alternatively, the peptide composition may have a weight-average molecular weight of at least 500 Da, 600 Da, 700 Da, 800 Da, 900 Da, 1000 Da, 1100 Da, 1200 Da, 1300 Da, 1400 Da, 1500 Da, 1750 Da, 2000 Da, 2250 Da, 2500 Da, 2750 Da or 3000 Da. In addition or alternatively, the peptide composition may have a weight average molecular weight of at most 3500 Da, 3000 Da, or 2500 Da, or 2250 Da, or 2000 Da, or 1750 Da, or 1700 Da, or 1650 Da or 1600 Da.

A preferred way of measuring the (average) molecular weight and/or molecular weight distribution (from which the molecular weight distribution can also be derived), of the collagen hydrolysate is by high performance size exclusion chromatography (HPSEC). The following protocol is a preferred HPSEC protocol:
The Agilent HPLC, 1260 Infinity series (G1316A, G1329B, G1311C, G1315D) with a TSKgel SWXL precolumn und a G2000SWXL column (Tosoh Bioscience) is used. Analysis is performed with the WinGPC software (PSS). The eluent is 100 mM phosphate buffer pH 5.3. Samples are eluted from the column (e.g. 0.5 mL/min, isocractic) and monitored with UV detection (e.g. 214 nm, analysis time: 40 min per injection + 180 min equilibration). Calibration is performed with the Narrow Calibration Standard (Low FILK).

Without being bound by theory, the present inventors consider that the relative amounts of cyclic peptides (especially cGP) and other peptides in the peptide composition (especially gly-pro-x, gly-pro-hyp, gly-pro-ala) contribute to its biological activity, although further collagen dipeptides may also contribute thereto.

In embodiment, the peptide composition comprises 1.0x10² - 1.0x10⁵ ppm total gly-pro-x total tripeptides and/or total tripeptides. In an embodiment, the peptide composition comprises 5.0x10² - 1.0x10⁴ ppm gly-pro-x tripeptides and/or total tripeptides. In an embodiment, the peptide composition comprises 1.0x10³ - 8.0x10³ ppm gly-pro-x tripeptides and/or total tripeptides. In embodiment, the peptide composition comprises 3.0x10³ - 6.0x10³ ppm gly-pro-x tripeptides and/or total tripeptides. In an embodiment, the peptide composition comprises 2.0x10³ - 5.0x10³ ppm gly-pro-x tripeptides and/or total tripeptides.

In embodiments of the invention, the peptide composition comprises 1.0x10³ - 2.0x10⁵ ppm, preferably 1.0x10⁴ - 1.0x10⁵ ppm, more preferably 2.0x10⁴ - 8.0x10⁴ ppm cGP, even more preferably 4.0x10⁴ - 6.0x10⁴ ppm cGP.

In embodiments of the invention, the peptide composition comprises 5.0x10² - 1.0x10⁴ ppm, preferably 2.0x10³ - 8.0x10³ ppm, more preferably 3.0x10³ - 7.0x10³ ppm, even more preferably 4.0x10³ - 6.0x10³ ppm gly-pro-hyp tripeptide.

In an embodiment, the peptide composition of the present invention comprises at least 0.1 ppm glycine-proline-hydroxyproline tripeptide, or at least 1 ppm glycine-proline-hydroxyproline tripeptide, or at least 5 ppm glycine-proline-hydroxyproline, or at least 10 ppm glycine-proline-hydroxyproline. In addition or alternatively, in embodiments the peptide composition of the present invention comprises no more than 10000 ppm glycine-proline-hydroxyproline, or no more than 7500 ppm glycine-proline-hydroxyproline, or no more than 5000 ppm glycine-proline-hydroxyproline. In a preferred embodiment, the peptide composition of the present invention comprises 1 - 1000 ppm glycine-proline-hydroxyproline tripeptide, preferably 5-500 ppm glycine-proline-hydroxyproline tripeptide, more preferably 10 - 100 ppm glycine-proline-hydroxyproline tripeptide. In a preferred embodiment, the peptide composition of the present invention comprises 0.1 - 8.0x10³ ppm, preferably 1 - 7.0x10³ ppm glycine-proline-hydroxyproline tripeptide.

In embodiments of the invention, the peptide composition comprises 1.0x10¹ - 1.0x10⁴ ppm, preferably 2.0x10¹ - 0.5x10⁴ ppm, more preferably 5.0x10¹ - 1.0x10³ ppm, even more preferably 1.0x10² - 5.0x10² ppm gly-pro-ala tripeptide.

In embodiments of the invention, the peptide composition comprises 0.1 - 50 ppm, preferably 0.5-20 ppm, more preferably 1-10 ppm pro-gly-pro tripeptide. In embodiments of the invention, the peptide composition comprises 0.1 - 50 ppm, preferably 0.5-20 ppm, more preferably 1-10 ppm pro-hyp-gly tripeptide.

In various embodiments, the peptide composition comprises hydroxylysine in an amount of 100-2500 ppm, preferably 200-2000 ppm. The amount of hydroxylysine in the peptide composition may be preferably 300-1500 ppm, more preferably 400-1000 ppm. In various embodiments, the peptide composition comprises hydroxyproline in an amount of 1000-50000 ppm, preferably 2000-25000 ppm. The amount of hydroxyproline in the peptide composition may be preferably 5000-30000 ppm, more preferably 10000-20000 ppm, even more preferably 14000-18000 ppm.

Preferably, the peptide composition is characterized by a cGP:Gly-Pro-Hyp weight ratio of 1:1 - 1000:1, preferably 10:1 - 100:1, calculated on total weight of peptides in the peptide composition. Preferably, the peptide composition is characterized by a cGP:Gly-Pro-Ala weight ratio of 10:1 - 10000:1, preferably 100:1 - 1000:1. Preferably the peptide composition is characterized by a cGP:(Gly-Pro-Hyp+gly-pro-ala) weight ratio of 1:1 - 1000:1, preferably 10:1 - 100. Preferably, the peptide composition is characterized by a cGP:Gly-Pro-x weight ratio of 1:1 - 1000:1, preferably 10:1 - 100:1. Preferably, the peptide composition is characterized by a Gly-Pro-Hyp:Gly-Pro-Ala weight ratio of 1:1 - 1000:1, preferably 10:1 - 100:1. Preferably, the peptide composition is characterized by a cGP:total linear dipeptides weight ratio of 1:1 - 10000:1, preferably 1:10 - 1:1000. Preferably, the peptide composition is characterized by a total linear dipeptides:total linear dipeptides weight ratio of 10:1 - 1:10, preferably 5:1 - 1:5, more preferably 2:1 - 1:2.

In embodiments of the invention, the peptide composition comprises 0.1 - 100 ppm, preferably 0.5-50 ppm, more preferably 1-20 ppm, even more preferably 2-15 ppm pro-hyp dipeptide. In embodiments of the invention, the peptide composition comprises 0.1 - 200 ppm, preferably 0.5-100 ppm, more preferably 1-50 ppm, even more preferably 5-30 ppm hyp-gly dipeptide. In embodiments of the invention, the peptide composition comprises 1.0x10⁻¹ (i.e. 0.1)- 1.0x10⁴ ppm, preferably 1.0x10¹ (i.e.10)- 0.5x10⁴ ppm pro-gly dipeptide. The amount of pro-gly dipeptide is more preferably 1.0x10² - 1.0x10³ ppm or 2.0x10² - 8.0x10² ppm. In embodiments of the invention, the peptide composition comprises 1.0x10¹ - 5.0x10⁴ ppm, preferably 1.0x10² - 1.0x10⁴ ppm gly-pro dipeptide. The amount of gly-pro dipeptide is more preferably 5.0x10² - 8.0x10° ppm or 1.0x10³ - 4.0x10³ ppm. In embodiments of the invention, the peptide composition comprises 0.1 - 100 ppm, preferably 0.5-50 ppm ala-pro dipeptide. The amount of ala-pro dipeptide is more preferably 1-20 ppm or 2-40 ppm.

In embodiments of the invention, the peptide composition comprises an amount of collagen dipeptides of less than 5.0x10⁴ ppm, preferably less than 1.0x10⁴ ppm. The amount of collagen dipeptides can be less than 5.0x10³ ppm or less than 3.0x10³ ppm. The amount of collagen dipeptides can for example be 1.0x10² - 2.0x10⁴ ppm. More preferably, the amount of collagen dipeptides is 0.5x10³ - 2.0x10⁴ or 1.0x10³ - 1.0x10⁴ ppm or 2.0x10³ - 5.0x10³ ppm.

The skilled person knows how to convert "ppm" values to "mg/kg", "mg/g" or "wt.%" values with respect to the concentration of peptides in a peptide composition. For solids, 1 ppm equals 1 mg of substance per kg of solid (mg/kg). A value of 1000 ppm of peptide X in a peptide composition therefore equals 1000 mg/kg or 1 mg/g or 0.1 wt.%, calculated on total weight of peptides in the peptide composition.

In embodiments of the invention, the peptide composition comprises 0-5%, preferably 0.1-2%, more preferably 0.2-1% by weight of peptides with a molecular weight of at most 250 Da. In embodiments of the invention, the peptide composition comprises 1-25%, preferably 5-15%, more preferably 7-13% by weight of peptides with a molecular weight of 250-450 Da. In embodiments of the invention, the peptide composition comprises 1-25%, preferably 5-15%, more preferably 7-13% by weight of peptides with a molecular weight of at most 450 Da.

In embodiments of the invention, the peptide composition comprises 10-40%, preferably 15-35%, more preferably 20-30% by weight of peptides with a molecular weight of 450-1000 Da. In embodiments of the invention, the peptide composition comprises 2-30%, preferably 5-20%, more preferably 7-15% by weight of peptides with a molecular weight of 0-500 Da or at most 500 Da.

In embodiments of the invention, the peptide composition comprises 10-40%, preferably 15-35%, more preferably 20-30% by weight of peptides with a molecular weight of 500-1000 Da. In embodiments of the invention, the peptide composition comprises 20-70%, preferably 35-50% by weight of peptides with a molecular weight of 1000-2000 Da.

In embodiments of the invention, the peptide composition comprises 10-40%, preferably 15-30% by weight of peptides with a molecular weight of 2000-5000 Da.

In embodiments of the invention, the peptide composition comprises 0.1-10%, preferably 0.5-5% by weight of peptides with a molecular weight of 5000-10000 Da.

In embodiments of the invention, the peptide composition comprises at most 2%, preferably 0.01-2%, more preferably 0.1-1% by weight of peptides with a molecular weight more than 10000 Da, calculated on total weight of total peptides in the peptide composition.

Preferably, the peptide composition comprises 10-60%, more preferably 20-40% by weight of peptides with a molecular weight below 1000 Da.

The peptide composition according to the invention preferably has a polydispersity of 1.3-1.9, more preferably 1.4-1.8, even more preferably 1.5-1.7

The peptide composition obtainable by the method may be subjected to processes such as filtration, centrifugation, concentration, ultrafiltration, lyophilization, and/or powdering, if desired. The peptide composition is preferably provided as a dried powder.

The peptide composition of the invention is preferably a collagen peptide composition. In addition or alternatively, in an embodiment the peptide composition of the invention is entirely obtained from collagen, more preferably a collagen hydrolysate.

### Use of the peptide composition

Oxidative stress may occur when there is an imbalance between the production of reactive oxygen species (ROS) and the body's ability to neutralize them with antioxidants. ROS are highly reactive molecules that can damage cellular components such as proteins, lipids, and DNA. It is generally believe that this damage can trigger inflammation, neuro-inflammatory responses and/or responses activating fibrogenic pathways. Chronic inflammation is also considered a key driver of fibrosis by promoting the activation of fibroblasts.

The in vitro 2,2-diphenyl-1-picrylhydrazyl (DPPH) assay measures the ability of a substance or composition to neutralize free radicals, specifically the DPPH radical, and assesses its antioxidant activity. The DPPH assay is used in the field of biochemistry, pharmacology, and food science to screen and evaluate the antioxidant potential of various substances compositions.

The presents inventors demonstrated with the DPPH assay that cGP and further peptides in the peptide composition of the invention have surprising synergistic anti-oxidative and anti-inflammatory properties, encompassing also anti-neuroinflammatory properties. In particular, synergistic and anti-oxidative and anti-inflammatory effects were observed for the combination of cGP with glycine-proline- hydroxyproline and glycine-proline-alanine tripeptide. Without being bound by theory, the present inventors consider that further collagen dipeptides may also contribute to the anti-oxidative properties. In addition or alternatively, the combination of the various peptides may together provide highest health benefits.

Various embodiments of the invention are exemplified by a peptide composition disclosed herein, for use as a medicament.

In an embodiment, the peptide composition as disclosed herein is an active ingredient.

Described is a use of the peptide composition disclosed herein for reducing oxidative stress. In preferred embodiments, the disclosure pertains to a peptide composition disclosed herein, for use preventing and/or treating oxidative stress. The peptide composition in addition or alternatively is useful in preventing and/or treating resulting conditions or symptoms associated with oxidative stress, such as, but not limited to inflammatory conditions (e.g. rheumatoid arthritis, inflammatory bowel disease), fibrosis, infection, autoimmune disease (e.g. rheumatoid arthritis, thyroid disease), neurological conditions, cancer, kidney diseases (e.g. chronic kidney disease, nephropathy), atherosclerosis, or eye diseases (e.g. age-related macular degeneration, cataract).

The term "preventing and/or treating" in the context of the current invention can also be encompassed by the term "ameliorating", which means encompasses both the "preventing" and the "treating" of a condition. As used herein, "ameliorating" also encompasses "curing".

The term "oxidative stress" in the context of the present disclosure means a condition characterized by an excess of oxidants and/or a decrease in antioxidant levels. Cellular oxidants may include, but are not limited to, one or more of: radicals of oxygen (superoxide anion, hydroxyl radical, and/or peroxy radicals); reactive non-radical oxygen species such as, for example, hydrogen peroxide and singlet oxygen; carbon radicals; nitrogen radicals; and sulfur radicals. The condition of oxidative stress may result in, for example, cellular damage, impaired performance of cells and/or cell death.

Described is a use of the peptide composition disclosed herein for prevention and/or treating inflammation and/or diseases that are characterized by an inflammatory component, including, but are not limited to inflammation (e.g. neuroinflammation), fibrosis, diseases characterized by an acute inflammation caused by a bacterial or viral infection e.g. meningitis, sepsis, malaria or chronic inflammation such as rheumatoid arthritis, osteoarthritis, psoriasis, chronic bronchitis, chronic obstructive pulmonary disease, inflammatory bowel disease (ulcerative colitis and Crohn's disease) and multiple sclerosis.

In preferred embodiments, the disclosure pertains to a use of the peptide composition disclosed herein for reducing inflammation.

Antioxidants can play a role in mitigating inflammation by neutralizing reactive oxygen species (ROS) and free radicals, which are often associated with inflammatory processes. It is considered by present inventors that the peptide composition is particularly suitable for reducing inflammation and treatment of inflammatory conditions. In particular, oxidative stress is implicated in various neurological conditions, in particular neuro-inflammatory conditions and/or neurodegenerative diseases. Accumulating evidence suggests that mitochondrial dysfunction and oxidative stress play especially major roles in aging and age-dependent neurological disorders. Without being bound by theory, the compounds with anti-oxidative and/or anti-inflammatory effects are considered useful to treat these conditions including by targeting the underlying cause.

Described is the peptide composition (obtainable by the method) disclosed herein for the manufacture of a medicament, preferably a medicament for preventing and/or treating a condition disclosed herein. In a preferred embodiment, the present disclosure pertains to the peptide composition for the manufacture of a medicament for preventing and/or treating a condition selected from the group consisting of oxidative stress, inflammation, a neurological condition and fibrosis.

Described is a method of preventing and/or treating a subject, comprising administering the peptide composition (obtainable by the method) disclosed herein to said subject. The method preferably relates to a method for the prevention and/or treatment of an individual suffering from a condition disclosed herein, such as a subject suffering a condition selected from the group consisting of oxidative stress, inflammation, a neurological condition and fibrosis. The method preferably relates to administering an amount of the peptide composition effective in preventing and/or treating said condition.

### Use in ameliorating fibrosis

Wound healing is a dynamic and multi-stage process necessary to reconstruct and regenerate the tissue after damage. The wound healing process can be classified into the four distinct phases:
(1) hemostasis, involving vasoconstriction, primary hemostasis, and secondary hemostasis, and that stops the bleeding;
(2) inflammatory phase, in which damaged and dead cells, along with pathogens or debris, are cleared out;
(3) proliferative phase, in which new tissue formation and angiogenesis occurs;
(4) remodelling phase, wherein appropriate tensile strength is achieved through reorganization, degradation, and resynthesis of the extracellular matrix (in particular collagens).

An abnormal or exuberant response in one or more of the wound healing phases can lead to an undesirable amount of pathological fibrous tissue formation, referred to as fibrosis or scar tissue formation. The activation of fibroblastic or highly contractile myofibroblasts is thought to be a major contributor to the accumulation of excessive and stiff scar tissue as seen in pathological fibrotic conditions. Although mild fibrosis to a certain extent can be part of the normal tissue replacement process, excessive fibrosis can lead to physical, along with significant psychological, emotional, and social problems. Several examples of the negative impact of excessive fibrosis are illustrated herein.

First, fibrosis can manifest as an organ-specific condition. Organ-specific fibrosis can have different underlying reasons, but is often associated with inflammation and/or occurring as a symptom of underlying disease. Unchecked fibrosis can result in destruction of the architecture of the underlying organ and impairment of organ function. Organ-specific fibrotic disorders include, for example pulmonary fibrosis, pulmonary hypertension, asthma, chronic obstructive pulmonary disease, liver fibrosis, kidney fibrosis, nonalcoholic steatohepatitis (NASH), musculoskeletal fibrosis, atrial fibrosis, Dupuytren's contracture, cellulite, endometriosis, keloid formation in the connective tissue and the like.

Second, fibrosis can occur after tissue manipulation resulting from surgery, i.e. in the form of post-operative fibrosis. For example, post-operative fibrosis is a typical problem seen following abdominal, neurological, spinal, vascular, thoracic or other types of surgery using both classical open and arthroscopic/laparoscopic procedures. Post-operative fibrosis can negatively influence the result of surgery and healing. For example, glaucoma surgery is a challenging procedure and not performed widespread clinically because of the severe complications that can arise from excessive fibrosis. Postoperative adhesions (PA) are examples of fibrotic tissues that are the most common driver of long-term morbidity after for instance abdominal, thoracic and pelvic surgery.

Third, fibrosis can occur around a medical implant and/or device, e.g. due to trauma, abnormal inflammation/infection, a foreign body response and/or implant/device (micro)movement. In that case, the fibrosis can also be referred to as implant-related fibrosis. For instance, breast implants and methods for breast reconstruction and augmentation are well known and have been used for a period of over twenty years. However, the formation of excess fibrosis remains an important issue associated with breast implants. The excessive fibrosis may lead to pathological tightening around the implant, also known as capsular contracture. Capsular contraction can lead to an implant that is misshapen, painful, hard and can attain an unnatural appearance and feel. Unwanted fibrosis around implants is also typically seen for implantable sensors, drug delivery devices, pacemakers, cardiovascular stents, and orthopedic implants, to name a few. The encapsulation of such implants by a fibrotic membrane can "shield off" the implant from the body, thus preventing adequate tissue integration and ultimately necessitating implant removal.

Fourth, skin-related fibrosis can occur as a result of acute injury or irritation to the skin, and which mostly can be classified as being either hypertrophic scars or keloids. The difference between hypertrophic scars and keloids lies in that hypertrophic scars typically develop within the original wound boundaries and are prone to regress over time, while keloids typically grow without limitation and rarely regressing. Hypertrophic scars or keloids frequently are the result of cuts, burns, acne scars, chickenpox scars, piercings, scratches, surgical cuts, tattoos, tattoo removal, insect bites, and vaccination sites, to name a few. The mechanisms of formation of hypertrophic scar and keloids are not yet completely understood, however it is believed that inflammation is involved in modulating collagen synthesis, since the degree of inflammation tend to positively correlate to final scar size.

Finally, fibrosis is a common symptom resulting from medical conditions such as fatty liver disease, chronic obstructive pulmonary disease, asthma, or systemic sclerosis.

There are several anti-fibrotic drugs known that can play a role in reducing excessive fibrosis. These include for lung fibrosis-approved drug Pirfenidone or pan-αv integrin inhibitors for lung fibrosis. A limitation of these drugs is that they require approval from federal agencies and medical prescription, and are associated with several adverse effects. For example, Pirfenidone reduces fibrosis by downregulating the production of growth factors and procollagens I and II. However, Pirfenidone is also associated with gastrointestinal, skin, and hepatic side effects. Other, non-pharmaceutical, anti-fibrotic products including both traditional plant-based materials and protein, however, there currently is no strong evidence to support their efficacy. Overall, the currently-known anti-fibrotic products appear insufficiently effective particular with regard to limiting tissue contraction and activation and/or transdifferentiation in skin cells and/or stromal cells such as myofibroblasts.

There remains an unmet need for active ingredients that ameliorate fibrosis, in particular more effectively and/or safely. Foremost, there is a need for active ingredients that can prevent fibrosis, e.g. by reducing cellular contraction, activation and/or transdifferentiation in skin cells and/or stromal cells such as myofibroblasts, by ameliorating oxidative stress and/or by ameliorating inflammation, in particular as implant coatings with myofibroblast-suppressing and contraction-supressing features.

Present inventors found that the peptide composition disclosed herein may be particularly effective in ameliorating fibrosis. Without being bound by theory, the mechanism of action is considered to involve the prevention of the contraction, activation, transdifferentiation and/or collagen (type I) overproduction in skin cells and/or stromal cells (e.g. fibroblasts and myofibroblasts).

The present findings by the inventors are surprising, because cGP has been suggested in the art (JP2019206481A) as a promoter of production of type I and type V collagen and therefore suggested as a suitable skin anti-aging agent. Considering that fibrosis is characterized by excess collagen synthesis (mostly collagen type I), this points to the direction that cGP would worsen fibrosis. However, the current inventors unexpectedly found that cGP, in conjunction with other peptides, may allow for collagen synthesis but at the same time inhibit excessive collagen synthesis as a hallmark of fibrosis. Without being bound by theory, providing for a balanced collagen synthesis may be in part a mechanism by which cGP in conjunction with other peptides effectively prevent and/or treat fibrosis.

Embodiments of the invention include a peptide composition disclosed herein, for use in preventing and/or treating fibrosis. The peptide composition may ameliorate excessive fibrosis in one of the aforementioned examples, thus providing an alternative to anti-fibrotic drugs described herein.

The term "fibrosis" in the context of the current invention means a condition in which there is abnormal production, accumulation, and/or deposition of extracellular matrix (proteins) such as by skin cells and/or stromal cells, in particular fibroblasts and/or myofibroblasts. For example, an "abnormal production" means that the production of extracellular matrix (proteins) exceeds the degradation rate. The term "fibrosis" encompasses an abnormal contraction of extracellular matrix (proteins). For example, an "abnormal contraction" means that the contraction is too high, so that the tissue is overly stiff and such that it is associated with negative functional and/or cosmetic effects. Typically, contraction occurs during the proliferative phase, during which a cell-rich granulation tissue is formed. The skilled person is aware that contraction is an important part of wound healing, as it enables wound closure. However, wound contraction in has both positive and negative effects. It is beneficial to wound healing by narrowing the wound margins, which leads to wound closure; however, excessive contraction causes formation of undesirable contracture and scarring, leading to cosmetic and functional problems (Li et al. J Tissue Viability. 2011 Nov; 20(4): 108-120).

There are different methods that are capable of measuring presence of fibrosis (and the inhibition thereof) in the context of the current invention, which encompass *in vitro* and *in vivo* methods. Suitable *in vitro* models include the hydrogel contraction assay and/or the myofibroblast transformation assay as disclosed herein in the Examples. A suitable *in vivo* model is the subcutaneous or intraperitoneal implantation model as disclosed by Farah et al. (Nat Mater. 2019 August; 18(8): 892-904), for instance to study efficacy of peptide-loaded and/or - coated implants.

Suitable models to study fibrosis include models that have been developed to asses the amount of foreign body response (FBR) *in vitro* or *in vivo.* For example, Sharifi et al. (Adv Healthc Mater. 2019 Feb;8(4):e1801425) have developed an *in vitro* FBR-on-a-chip model as a physiologically relevant *in vitro* setting which reproduces the innate immune cell interactions with implants. The FBR-on-a-chip model is a suitable model to study fibrosis in the context of the current invention.

The prevention and/or the treatment of the fibrosis can in the context of the current invention be of therapeutic or non-therapeutic, depending on the severity of fibrosis and/or the context. The professional medical practitioner is able to determine on a case-by-case basis whether the fibrosis is pathological or in a pathological subject, or non-pathological or in a healthy subject. Therapeutic use can for example mean a use in severe fibrosis and/or pathological situation and/or where there are symptoms of pain and suffering which may lead to (serious) health and psychological risks. Non-therapeutic use can for example mean a use fibrosis having a nature or severity such that there are no symptoms of pain and suffering and/or it naturally disappears over time (i.e. it is not chronic). A non-therapeutic use in particular encompasses a cosmetic use e.g. to improve the appearance of the skin affected by fibrosis, but in a non-pathological context and/or in absence of symptoms of pain and suffering.

In a preferred embodiment, the fibrosis is one or more selected from the group consisting of medical implant-related fibrosis, surgery-related fibrosis, adhesion formation including post-surgical adhesion formation, skin fibrosis, a hypertrophic scar, a keloid, liver fibrosis, pulmonary fibrosis, pancreatic fibrosis, kidney fibrosis, vascular fibrosis, endometriosis, atrial fibrosis and heart fibrosis.

In an preferred embodiment, the fibrosis is organ-specific fibrosis. The organ-specific fibrosis is preferably one or more selected from the group consisting of pulmonary fibrosis, liver fibrosis, pancreatic fibrosis, kidney fibrosis, musculoskeletal fibrosis, atrial fibrosis, heart fibrosis, Dupuytren's contracture, cellulite, endometriosis and keloid formation in the connective tissue.

In a preferred embodiment, the fibrosis is a symptom in a condition selected from the group consisting of pulmonary hypertension, fatty liver disease, Dupuytren's contracture, cellulite, chronic obstructive pulmonary disease, nonalcoholic steatohepatitis (NASH), asthma and systemic sclerosis.

In an embodiment, the fibrosis as disclosed herein is not the condition of cystic fibrosis and/or the peptide composition is not for the prevention and/or treatment of the condition of cystic fibrosis. Cystic fibrosis is a genetic condition that typically causes damage and scarring in different organs and may necessitate treatment of thick mucus formation and infection. Nevertheless, the peptide composition may prevent and/or treat the fibrosis as a symptom of cystic fibrosis (without targeting the disease itself). Hence, in an embodiment, the fibrosis is fibrosis (as symptom) in the condition cystic fibrosis.

In an embodiment, the fibrosis as disclosed herein is not the condition of myelofibrosis and/or the peptide composition is not for the prevention and/or treatment of the condition myelofibrosis. Myelofibrosis is a bone marrow blood cancer, wherein healthy marrow is typically replaced by scar tissue. Prevention and/or treatment of myelofibrosis may necessitate a therapy that targets the cause of these disease and/or the underlying myeloproliferative condition. Nevertheless, the peptide composition may prevent and/or treat the fibrosis as a symptom in myelofibrosis (without targeting the disease itself). Hence, in an embodiment, the fibrosis is fibrosis (as symptom) in the condition myelofibrosis.

The present inventors found a dose-response effect of the peptide composition of the invention in ameliorating fibrosis. Hence, the current invention pertains also to a most optimal amount of the peptide composition for achieving a highest effect in preventing and/or treating fibrosis.

Without being bound by theory, there may be different mechanisms that alone or in conjunction lead to the beneficial anti-fibrosis effects.

The present inventors found that the peptide composition is surprisingly effective in preventing fibrosis, at least in part by preventing the contraction, activation and/or transdifferentiation in skin cells and/or stromal cells, particularly fibroblasts and/or myofibroblasts. In particular, it was found that the peptide composition can ameliorate the (trans)differentiation of skin cells (e.g. fibroblasts) and/or stromal cells into myofibroblasts.

Moreover, the present inventors also found that the peptide composition inhibits an overproduction of collagen and/or provides for a balanced collagen synthesis (e.g. in skin cells and/or stromal cells), foremost seen for collagen type I.

Excess collagen production is typically a hallmark in fibrosis. The cells responsible for collagen production in fibrosis are believed to be fibroblasts and myofibroblasts. For instance, lung fibrosis and other types of fibrosis typically favor a predominance of at least type I and/or type III collagen, but also collagen type V and other types of collagen can be overexpressed in fibrosis. There often additionally is an increase in other extracellular matrix proteins such as fibronectin, elastin, laminin, hyaluronan, chondroitin sulfate and/or heparin sulfate proteoglycans.

Without being bound by theory, providing for a balanced collagen synthesis may be in part a mechanism by which the peptide composition of the invention is highly effective in ameliorating (in particular preventing) fibrosis, without counteracting the normal wound healing (by not completely bringing collagen synthesis to a halt).

In a preferred embodiment, the preventing and/or treating fibrosis is by ameliorating, preferably decreasing, cell activation. In a preferred embodiment, the preventing and/or treating fibrosis is by ameliorating, preferably decreasing, cell transdifferentiation. In a preferred embodiment, the preventing and/or treating fibrosis is by ameliorating, preferably decreasing, overproduction of collagen.

In an embodiment, the peptide composition reduces or increases the migration of a cell, preferably a skin cell and/or a stromal cell. In an embodiment, the peptide composition reduces or increases proliferation of a cell, preferably a skin cell and/or stromal cell. In an embodiment, the peptide composition reduces or increases the (trans)differentiation of a cell, preferably a skin cell and/or a stromal cell. In an embodiment, the peptide composition reduces or increases the activation of a cell, preferably a skin cell and/or a stromal cell. In an embodiment, the peptide composition reduces or increases the contraction of extracellular matrix, a skin cell and/or a stromal cell. In an embodiment, the peptide composition reduces or increases wound contraction.

In a preferred embodiment, the peptide composition ameliorates, preferably reduces, the (trans)differentiation of a skin cell (e.g. fibroblast) and/or a stromal cell into a myofibroblast.

In an embodiment, a reduced contraction means a reduction in at least 5%, preferably at least 10%, more preferably at least 20%, even more preferably at least 30%, most preferably at least 50%, e.g. compared to a negative control group (not receiving the treatment). A reduction in contraction is preferably measured in the collagen hydrogel contraction assay as disclosed herein.

In an embodiment, a reduced differentiation and/or transdifferentiation means a reduction in at least 5%, preferably at least 10%, more preferably at least 20%, even more preferably at least 30%, most preferably at least 50%, e.g. compared to a negative control group (not receiving the treatment). A reduction in differentiation and/or transdifferentiation is preferably measured in the myofibroblast transformation assay as disclosed herein.

In an embodiment, the peptide composition reduces or inhibits the overexpression of one or more extracellular matrix proteins, preferably one or more collagens, more preferably one or more of collagen type I, III and/or V (e.g. in a skin cell and/or stromal cell). In an embodiment, the peptide composition promotes a balanced synthesis of one or more extracellular matrix proteins, preferably one or more collagens, more preferably one or more of collagen type I, III and/or V (e.g. in a cell such as a skin cell and/or stromal cell).

The term "skin cell" in the context of the current invention means any type of cell that is predominantly found in the skin (particularly epidermis) any type of cell that provides a specialized function in the skin (particularly epidermis), and/or any type of cell that forms the skin (particularly epidermis), and encompasses keratinocytes, melanocytes, Langerhans cells, and Merkel cells. The term "stromal cell" in the context of the current invention means any multipotent or pluripotent cell capable of developing specifically into a distinct type of connective tissue cell. The stromal cell may be a fibroblast, myofibroblast, smooth muscle cell, pericyte, or mesenchymal stem/stromal cell. The term "myofibroblast" in the context of the current invention means a cell type that is in between a fibroblast and a smooth muscle cell in differentiation. The "myofibroblast" may be formed for instance by one or more of the following pathways: partial smooth muscle differentiation of a fibroblastic cell, activation of a stellate cell, loss of contractile phenotype of a smooth muscle cell, direct myofibroblastic differentiation of a progenitor cell resident in a stromal tissue, homing and recruitment of a circulating mesenchymal precursor which can directly differentiate as above or indirectly differentiate through the other cell types as intermediates and/or epithelial to mesenchymal transdifferentiation of an epithelial cell. In a preferred embodiment, the myofibroblast is a cell that is positive (e.g. shown by staining) for the markers intermediate filament vimentin, α-smooth muscle actin and for paladin.

### Coatings, immobilization

A known strategy to reduce fibrosis around medical implants is to coat (e.g. immobilise) their surface with anti-fibrotic molecules in an attempt to reduce excessive fibrosis. These include coatings based on anti-inflammatory drugs, anti-fibrotic drugs, anti-proliferative drugs, and/or pro-angiogenic drugs to prevent excessive cellular fibrosis encapsulation. For instance, US6110155A describes a medical catheter carrying the anti-inflammatory corticosteroid dexamethasone, as a means of preventing tissue fibrosis. However, anti-inflammatory agents are preferably avoided, in order not to negatively influence normal wound healing and to avoid common side effects of corticosteroids and other anti-inflammatory agents. Advantageously, in view of the aforementioned limitations, the peptide composition disclosed herein appears particularly suitable to prevent fibrosis around a medical implantable, e.g. to thereby prevent and/or treat medical implant-related fibrosis.

Described is a substrate coated with the peptide composition disclosed herein.

In an embodiment, the peptide composition is provided as a coating on a substrate. The substate is preferably a medical device such as a medical implant. The medical device is preferably selected from the group consisting of a suture material, a drug-eluting bead, a cardiovascular implant, a stent, a catheter, a pacemaker, an electrical lead, an implantable cardiac defibrillator, a nerve stimulator, a degradable implant, an interocular lense, an implantable drug delivery device, an implantable pump, an intra-uterine device, a surgical mesh implant, a breast implant and a prosthesis.

The term "medical implant" in the context of the current invention means a device or tissue that is placed inside or on the surface of the body. The medical implant can be prosthetics, intended to replace missing body parts. The medical implant can be intended to deliver medication (e.g. as a drug delivery device), monitor body functions, or provide support to organs and tissues. The term "medical implant" encompasses implants made from skin, bone or other body tissues. The "medical implant" in the context of the current invention can be placed permanently or it may be removed once no longer needed. For example, a stent or hip implant is typically intended to be permanent, but chemotherapy ports or screws to repair broken bones can be removed when they no longer needed. The term "medical device" in the context of the current invention means any instrument, machine, implant, intended to treat, cure, prevent, mitigate, diagnose disease, and includes any medical device classified as such by the Medical Device Regulation in the European Union and/or the U.S. Food and Drug Administration.

In various embodiments, the peptide composition is immobilized on the substrate. The term "immobilization" or "to immobilize" in the context of the current invention means that a molecule (e.g. peptide) is chemically linked to a substrate, wherein the linking may be directly (e.g. by direct chemical conjugation) or indirectly (e.g. through a linker moiety or following chemical functionalization of the substrate). The immobilization in the context of the current invention may be reversible or irreversible. In an embodiment, the peptide composition is not released from the substrate preferably following immobilization. The immobilization in the context of the current invention encompasses non-specific immobilization (i.e. leading to immobilized molecules oriented in a random fashion), such as non-covalent adsorption or covalent non-specific attachment.

The molecule or compound may directly immobilized to the substrate without requiring a linker. In addition or alternatively, one or more linker molecules may be provided as intermediate between the substrate and the molecule to be immobilized. The "linker" in the context of the current invention preferably means a chemical linker, providing one or more chemical bonds as disclosed herein. In an embodiment, the substrate is functionalized during or after immobilization of the molecule to be immobilized, e.g. such as when the substrate is considered chemically inert and/or lacks functional moieties for chemical conjugation. The term "functionalization" in the context of the current invention means to alter the physical and/chemical properties of a surface, preferably to provide functional groups allowing for improved immobilization of a molecule.

In various embodiments, the peptide composition is provided in an amount of 0.00001 - 10 g, preferably 0.0001 - 1 g, more preferably 0.001 - 0.1 g, even more preferably 0.01 - 0.1 g, wherein the amount is the average amount per cm² surface area of the substrate.

In various embodiments, the peptide composition is provided in a carrier material (i.e. carrier) on the substrate. The carrier material may for instance be a degradable material, allowing for more sustained and/or tuneable release of an embedded molecule. The carrier may for instance be one or more of a polymer film, microparticle, hydrogel, collagen gel, extracellular matrix gel, or mixtures thereof. Example of carrier materials suitable in the context of the current invention include carbohydrates and polysaccharides such as starch, cellulose, dextran, methylcellulose, and hyaluronic acid, proteins or polypeptides such as albumin, fibrin collagen, and gelatin, or combinations thereof.

In various embodiments, the substrate and/or carrier material may be fibrous, wherein the fibers can be for instance in the micron and/or nanometer diameter range such as 100 nm-50 µm, or 200 nm-10 µm, or 500 nm 5 µm or 1-2 µm.

In various embodiments, the peptide composition is provided in a carrier in an amount of for example 0.00001 - 25 wt.%, or 0.0001 - 10 wt.% or 0.005 - 1 wt.%, calculated on the weight of the carrier. Based on dose-response curves *in vitro,* the present inventors found that a preferred concentration for anti-fibrosis may be in the range of 0.0001 - 5 wt.%, preferably 0.001 - 1 wt.%, more preferably 0.01 - 0.1 wt.%, calculated on the weight of the carrier.

Preferably, the carrier is adapted and/or provides a release profile that lasts for at least 2 days, or 3 days, or 4 days, or 5 days, or 6 days, 1 week, or 2 weeks, or 3 weeks, or 4 weeks, or 6 weeks, or 8 weeks, or 12 weeks. Preferably, the carrier is adapted and/or provides a release profile that lasts for at most 12 weeks, or 8 weeks, or 6 weeks, or 4 weeks, or 3 weeks, or 2 weeks, or 1 week.

The skilled person knows how to adapt the carrier to accommodate the appropriate release rate and/or release profile, e.g. by selecting the appropriate polymer of mixture thereof, the concentration of said polymer, the thickness of the coating.

In an embodiment, the peptide composition is releasable from the substrate and/or the coating. In an embodiment, the substrate and/or the coating is configured for release of the peptide composition. In an embodiment, the release is a sustained release. In an embodiment, the release is according to a sustained release profile.

As used herein the terms "sustained release" or "sustained release profile" mean a release of substance of compound (e.g. peptide) gradually over an extended period of time (e.g. a period of several days, weeks, or months). In an embodiment, the amount released over an initial period is similar to or less than the amount released over the same period after several days or weeks. Preferably, the sustained release profile lasts at least 1 week, or 2 weeks, or 3 weeks, or 4 weeks, or 6 weeks, or 8 weeks, or 12 weeks.

In an embodiment, the release exhibits a substantially linear rate of release. The term "linear rate of release" in the context of the current invention means that the substance or compound (e.g. peptide) is released at a rate that does not vary by more than about 20% over the desired period of time, preferably by not more than 10%.

In an embodiment, the peptide composition is released for least 2 days, or 3 days, or 4 days, or 5 days, or 6 days, 1 week, or 2 weeks, or 3 weeks, or 4 weeks, or 6 weeks, or 8 weeks, or 12 weeks. In addition or alternatively, the peptide composition is released for at most 12 weeks, or 8 weeks, or 6 weeks, or 4 weeks, or 3 weeks, or 2 weeks, or 1 week.

In an embodiment, the peptide composition is released in an amount of 0.0001 g - 10 g, or 0.001 g - 1 g, or 0.01 g - 0.1 g per day on average.

The desired release rate, release profile and/or the amount released can vary depending on the bodily site and/or the application.

The "release" in the context of the current invention preferably means the *in vivo* release, i.e. following implantation. The *in vivo* release can be determined by implanting coated samples with known peptide content, harvesting samples at various time points, and then determining the amount of residual peptide (i.e. non-released peptide) in the samples. Based on these values, a release profile curve can be plotted. In addition or alternatively, the "release" can be determined *in vitro,* preferably under conditions that approximate or mimic the *in vivo* conditions (temperature, pH, aqueous medium, and/or presence of enzymes etc...) that may influence the release rate *in vivo.* A preferred *in vitro* protocol for measuring release involves incubating samples in phosphate buffered saline (PBS) at 37 °C, and collecting/refreshing the PBS with eluted peptide at different time points. The concentration of peptide in the released samples can be measured by any suitable method, such as HPLC. A suitable HPLC protocol for measuring cyclic glycine-proline involves using two eluents, A [0.1% (v/v) TFA/water] and B [0.1% (v/v) TFA/CAN], linearly increasing eluent B: 0% (0 - 15 min), 0% - 50% (15 - 45 min), 50% - 0% (45 - 50 min), and 0% (50 - 60 min). A suitable flow rate is 1.0 mL/min, and a suitable column temperature is 30°C. Elution can be monitored by absorbance at 214 nm. Concentrations are normalized against a calibration curve obtained by diluting stock solutions in PBS.

### Use in ameliorating neurological conditions/improving brain-related functions

Maintenance or improvement of neurological and brain functions is important to the general well-being throughout all stages of life. A decline of the neurological and brain functions typically leads to reduced daily functioning and overall health, e.g. due to the negative impact on several neurological functions such as neuro-motoric functions, neuro-sensory functions or cognitive functions (e.g. more specifically memory, mental flexibility and speed, learning). The decline of the neurological and brain functions is increasingly becoming apparent in the elderly as part of the ageing population and is recognized as a major health and social problem.

There are also several conditions that lead to pathologically impaired function of the nervous system and brain, examples thereof being neurodegenerative and/or neuroinflammatory conditions (e.g. Alzheimer's, Parkinson's), brain injury (e.g. cerebral ischemia), dementia, depressive disorders or neuropathy.

Because the efficiency of an individual's neurological and brain function can make all the difference in daily life, there is an unmet need for active ingredients suitable for ameliorating neurological conditions, either characterized an pathologically impaired function of the nervous system (e.g. due to a disease or disorder) or active ingredients which may simply lead to improvement of the neurological functions, such as by enhancing and/or preventing decline in the normal cognitive ability such as in terms of learning and memory.

Currently, the approach for improving neurological conditions is mostly by pharmacological intervention. For example, for treating Alzheimer's disease dementia, cholinesterase inhibitors such as donepezil (Aricept^{®}), galantamine (Razadyne^{®}, Razadyne ER^{®}, Reminyl^{®}, Nivalin^{®}) and rivastigmine tartrate (Exelon^{®}) are known to be efficacious. For treatment of neuropathy, cyclic antidepressants, such as amitriptyline and nortriptyline are prescribed. Strategies for improving cognitive function typically involves improving neurotransmitter level and signal transmission in neurotransmitter pathways which are believed to be involved in the process of learning ability and memory formation.

A major drawback of aforementioned pharmacological interventions is that they typically have side effects, which limits their widespread use and which in some cases lead to discontinuation of the therapy. In addition, not all of the aforementioned pharmacological interventions have been considered effective enough, hampering their widespread use.

The peptide composition disclosed herein presents a safe and effective alternative to aforementioned pharmacological interventions.

The D-galactose model is a well-recognized animal model to mimic the incremental loss of cognitive and motor performances in age-dependent neurological disorders (Parameshwaran et al. Rejuvenation Res. 2010 Dec; 13(6): 729-735). The D-galactose model is used to study diseases such as Alzheimer's disease, Parkinson's disease, cognitive decline, neuroinflammation, ischemic stroke, age-related changes in the central nervous system. The present inventors have shown that the peptide composition of the invention has the following surprisingly therapeutic benefits in the D-galactose model, although the effects do not appear to be limited to this list:
- increase in the spatial memory and learning,
- decrease in neuropathy / improved neuromotor (rotarod latency), neuromuscular (grip strength) and electrophysiological performances,
- decrease in neuroinflammation.

Embodiments of the invention include a peptide composition disclosed herein, for use in preventing and/or treating a (central) neurological condition, or a symptom resulting thereof, wherein the neurological condition, encompasses one or more selected from the group central nervous system inflammation and/or injury, movements conditions or disorders, neuromotor disease, neuromuscular disease, neurosensory disease, psychotic conditions or disorders, depressive conditions or disorders, anxiety conditions or disorders, nervous system and/or brain damage, cognitive conditions or disorders, personality conditions or disorders, mood conditions or disorders, migraine, epilepsy or convulsive disorders, neurodegenerative condition or disorder, autistic disorders, neuropathy, a neurodevelopmental disorder and neurotoxicity.

The central nervous system inflammation and/or injury may include one or more of concussive or traumatic injury and cognitive, learning or memory impairments resulting therefrom.

The movement condition or disorder disclosed herein can be one or more selected from the group consisting of Parkinson's disease, second Parkinsonism (e.g. postencephalitic Parkinsonism, Lewy body disease, degenerative diseases of the basal ganglia, extrapyramidal and movement disorders (e.g. including but not limited to tremor, essential tremor and drug-induced tremor, myoclonus, chorea and drug-induced chorea, drug-induced tics and tics of organic origin, drug-induced acute dystonia, drug-induced tardive dyskinesia, L-dopa-induced dyskinesia), neuroleptic-induced movement disorders (e.g. including but not limited to neuroleptic malignant syndrome (NMS), neuroleptic induced parkinsonism, neuroleptic-induced early onset or acute dyskinesia, neuroleptic-induced acute dystonia, neuroleptic-induced acute akathisia, cerebral palsy, neuroleptic-induced tardive dyskinesia, neuroleptic-induced tremor), restless leg syndrome and Stiff-man syndrome.

The psychotic condition or disorder disclosed herein can be one or more selected from the group consisting of schizophrenia (e.g. paranoid, disorganized, catatonic, undifferentiated or residual type), schizophreniform disorder, schizoaffective disorder (e.g. delusional or depressive type), delusional disorder, substance-induced psychotic disorder (e.g. psychosis induced by alcohol, amphetamine, cannabis, cocaine, hallucinogens, inhalants, opioids, or phencyclidine), personality disorders of the paranoid type and personality disorder of the schizoid type.

The anxiety condition or disorder disclosed herein may be one or more selected from the group consisting agoraphobia, generalized anxiety disorder (GAD), mixed anxiety and depression, obsessive-compulsive disorder (OCD), panic disorder, posttraumatic stress disorder (PTSD), social phobia and other phobias and panic attack.

Examples of nervous system and/or brain damage in the context of the current invention include but are not limited to cerebral ischemia, stroke, traumatic brain injury and spinal cord injury and spinal cord trauma.

The cognitive condition or disorder disclosed herein may be one or more selected from the group consisting of Alzheimer's disease, Parkinson's disease, delirium, substance-induced persisting delirium, dementia, dementia due to HIV disease, dementia due to Huntington's disease, dementia due to Parkinson's disease, dementia of the Alzheimer's type, dementia associated with beta-amyloid, behavioural and psychological symptoms of dementia, substance-induced persisting dementia and mild cognitive impairment.

The personality disorder disclosed herein may be one or more selected from the group consisting of obsessive-compulsive personality disorder and schizoid, schizotypal disorder.

The mood disorder disclosed herein may be one or more selected from the group consisting of bipolar disorders (I & II), cyclothymic disorder, depression, dysthymic disorder, major depressive disorder, treatment resistant depression, bipolar depression and substance-induced mood disorder.

The neurodegenerative condition disorder may be one or more selected from the group consisting of oxidative stress, such as Alzheimer's disease, Parkinson's disease, multiple sclerosis, amyotrophic lateral Sclerosis (i.e. Lou Gehrig's disease), Huntington's Disease and neurodegenerative lysosomal storage diseases.

The neuropathy as disclosed herein may be one or more selected from the group consisting of diabetic neuropathy, peripheral neuropathy, carpal tunnel, syndrome, alcoholic neuropathy, idiopathic neuropathy, Guillain-Barré Syndrome.

The neurodevelopmental disorder in the context of the present invention may be one or more selected from the group consisting of autism Spectrum Disorder (ASD), Intellectual Disability (ID), Specific Learning Disorder (SLD), a Communication Disorder (e.g. speech sound disorder, language disorder, and social communication disorder), a Motor Disorders (e.g. Developmental Coordination Disorder (DCD) and Tourette Syndrome) and Attention Deficit Hyperactivity Disorder (ADHD), preferably ADHD. In a preferred embodiment, the neurological condition disclosed herein is ADHD and/or the use pertains to ameliorating ADHD.

The invention is further illustrated by a peptide composition disclosed herein, for use in preventing and/or treating neuron, central nervous system and/or brain damage such as in frailty syndrome, aging, injuries, pathologies, diseases, infections and conditions causing and/or associated with an increased amount of central nervous system inflammation and/or central nervous system oxidative stress, or accelerating the recovery of central nervous system neuron or brain damage in individuals having injuries, pathologies (e.g. Amyotrophic Lateral Sclerosis and/or Multiple Sclerosis) and cognitive, learning or memory impairments resulting therefrom, diseases, infections and conditions causing and/or associated with an increased amount of central nervous system inflammation and/or central nervous system oxidative stress, and cognitive, learning or memory impairments resulting therefrom.

The uses of the invention disclosed herein may comprise administering to a subject in need thereof a therapeutically effective amount of a peptide composition as described herein, e.g. a method of treating.

In an aspect, the invention pertains to a use of a peptide composition as described herein for maintaining or improving cognition comprises, in particular for maintaining or improving of learning, plasticity and/or long term memory, wherein said use can be in a therapeutic and/or non-therapeutic context. In addition or alternatively, the use of the invention can pertain to maintaining and/or improving neurological and/or brain function, such as selected from the group consisting of memory, attention, concentration, alertness, mental flexibility and/or speed, learning, intelligence, language skills, problem solving capacity, consciousness, coping with psychological stress or tension, motivation, mobility, decision making capacity, reaction time and regulation of emotions, wherein said use can be in a therapeutic and/or non-therapeutic context. In embodiments, the maintaining and/or improving neurological and/or brain function can be associated with neurological dysfunction, condition, disorder such as disclosed herein.

The terms "cognition", "cognitive function" and "cognitive performance" can be used interchangeably in the context of the current invention and are related to any mental process or state that involves but is not limited to learning, memory, creation of imagery, thinking, awareness, reasoning, spatial ability, speech and language skills, language acquisition, capacity for judgment attention, alertness, mental flexibility and/or speed, coping with psychological stress or tension, decision making capacity and reaction time.

The composition as disclosed herein can have non-therapeutic and/or therapeutic use in the context of the current invention. Non-therapeutic use is illustrated by administering the composition of the invention in healthy subjects who do not receive therapeutic benefit, which in the context of the current invention typically means enhancing performance (e.g. cognitive function) starting from a normal healthy state. Therapeutic use is illustrated by administering the composition of the invention in pathological subjects and/or subjects who receive therapeutic benefit, such as by preventing and/or treating one or more of disease, health risk, psychological risks or symptoms of pain and suffering.

In an embodiment, the present invention pertains to a use of the composition in non-therapeutic improvement of cognitive function. In an embodiment, the cognitive function is selected from the group consisting of learning ability, memory, spatial memory, problem solving capacity, capacity for remembering, attention span, concentration, ability of language processing, spatial ability, speech, language skills, ability to focus, mental alertness and reaction time.

Embodiments of the invention are exemplified by non-therapeutic use of a peptide composition disclosed herein. Such embodiments include enhancing performance, e.g. cognitive function, starting from a normal healthy state and/or in otherwise healthy individuals. In preferred embodiments, the invention pertains to non-therapeutic use of the peptide composition in improving one or more cognitive functions, preferably selected from the group consisting of learning, memory, creation of imagery, thinking, awareness, reasoning, spatial ability, speech skills, language skills, language acquisition, capacity for judgment attention, alertness, mental flexibility, speed, coping with psychological stress and/or tension, decision making capacity and reaction time.

A non-therapeutic use of the composition of the invention may also pertain to general improvement of the control of muscles or improving neuro-motoric functions or neuro-sensory functions.

### Administration of the peptide composition

In preferred embodiments, the peptide composition (obtainable by the method) is provided as or is present in one or more formulations selected from the group consisting of food formulation, feed formulation, food supplement formulation, feed supplement formulation, or pharmaceutical formulation, preferably a food supplement formulation. In a preferred embodiments, the composition according to the invention is provided as or in a food supplement.

In preferred embodiments, the peptide composition (obtainable by the method) is provided in a formulation selected from the group consisting of a capsule, a tablet or a powder, preferably a powder.

In preferred embodiments, the peptide composition (obtainable by the method) is provided in a formulation selected from the group consisting of a drinkable solution or suspension, drink such as beer, syrup, artificially-flavoured drink, carbonated beverage, (water-soluble) powdered mixture, (water-soluble) paste, (water-soluble) powder, (water-soluble) tablet, (water-soluble) pill, (water-soluble) dragee, (water-soluble) caplet, (water-soluble) sachet, or (water-soluble) capsule. In addition or alternatively, the peptide composition as (obtainable by the method) may be present in a functional food such as a juice, shake, dairy drink, yoghurt, yoghurt drink, dessert, energy bar, nutritional bar, slimming bar, or confectionery such as gummies or centre-filled gummies.

In preferred embodiments, the peptide composition (obtainable by the method) is administered at a daily dose of 20 mg - 50 g, preferably 50 mg - 40 g, more preferably 0.1 g - 20 g, even more preferably 0.5 g - 10 g, yet even more preferably 1 g - 5 g, wherein the daily dose is the total dry weight amount of the composition. In a preferred embodiments, the peptide composition (obtainable by the method) is administered disclosed herein is administered at a unit dose of 10 mg - 25 g, preferably 25 mg - 10 g, more preferably 50 mg - 5 g, even more preferably 0.1 g - 1 g, wherein the unit dose is the total dry weight amount of the composition. In a preferred embodiment, the daily dose and/or unit dose is 0.1 g - 20 g or 0.5 g - 10 g.

The term "daily dose" as used herein refers to the total dry weight amount administered to a subject per day. The daily dose of the composition may be administered as a single unit dose, or as two, three, four or more unit doses. The two or more unit doses may be equal or different in amount. The daily dose of the composition as disclosed herein is preferably administered as two unit doses, more preferably as two unit doses each corresponding to 30-70%, preferably 40-60% of the daily dose amount. The term "unit dose" as used herein relates to the total dry weight amount administered to a subject in a single dose. The unit dose typically is in a pre-prepared form (e.g. prepacked dosage) ready for administration. The unit dose may for example (also) be identifiable from the product packaging or label.

In a preferred embodiments, the peptide composition (obtainable by the method) is administered to a subject repeatedly, preferably at least once every day or at least once every other day, and/or administered to a subject for at least two consecutive weeks, preferably for at least four consecutive weeks, more preferably for at least six consecutive week, most preferably for at least eight consecutive weeks.

The terms "consecutive" or "consecutively" used in the context of administration means that the administrations follow one another in order without gaps in a given time period. For example, when composition is administered "for 4 consecutive days", this means that the composition is administered for at least once a day for 4 days in a row (e.g. on Monday, Tuesday, Wednesday, and Thursday of the same week), irrespective of the number of administrations per day or the total number of administrations. For example, when a composition is administered "for 4 consecutive weeks", this means that the composition is administered for at least once a week for 4 weeks in a row, irrespective of the number of administrations per day, the number of administrations per week or the total number of administrations.

In a preferred embodiment, the peptide composition is administered orally, such as in the form of a food supplement.

In a preferred embodiment, the peptide composition is administered locally, such as parenterally, preferably subcutaneously and/or trans-dermally.

In an embodiment, the peptide composition is administered by injection.

In a preferred embodiment, the peptide composition is provided in a topical formulation. The topical formulation is preferably selected from the group consisting of a wound dressing, a hydrogel, a gel, an ointment, a plaster, a skin substitute, a tissue adhesive, a tissue sealant, a hemostat, a spray and a bandage. In various embodiments, the current invention pertains to a topical formulation comprising the peptide composition disclosed herein.

The peptide composition may be provided in the topical formulation in an amount of for example 0.00001 - 25 wt.%, or 0.0001 - 10 wt.% or 0.005 - 1 wt.%, calculated on the weight of the topical formulation. Based on dose-response curves *in vitro,* the present inventors found that a preferred concentration for anti-fibrosis may be in the range of 0.0001 - 5 wt.%, preferably 0.001 - 1 wt.%, more preferably 0.01 - 0.1 wt.%, calculated on the weight of the topical formulation.

In various embodiments, the current embodiment pertains to a non-therapeutic cosmetic use of the peptide composition disclosed herein. The cosmetic use can for example be for non-therapeutically improving skin appearance, such as due to scars, wrinkles, dyscolorations of the skin, skin irritation, volume augmentation, baldness, after skin peeling, dermabrasion and medical needling. The cosmetic use can be one or more of topical, intralesional, intraepithelial, intra-epidermal, intra-cutaneous, and subcutaneous administration into or onto the skin. In a preferred embodiment, the administration is topical administration onto the skin, e.g. wounded skin.

In various embodiments, the current invention pertains to a cosmetic composition comprising the peptide composition disclosed herein.

The peptide composition can be in a cosmetic composition, and therefore preferably further include acceptable excipients, lubricants and additives. The cosmetic composition may be in any form suitable for local application, for example, a solution, a gel, a solid, a paste anhydrous product, an emulsion obtained by dispersing an oil phase in water phase, an emulsion obtained by dispersing a water phase in an oil phase, a multi-emulsion, a suspension, a microemulsion, a microcapsule, microgranules, ionic (liposome) and non-ionic vesicle dispersants, a foam, and an aerosol or patch form further containing a compressed propellant. The cosmetic composition may be selected from the group consisting of a soap-type preparation, skin toner, nutrition lotion, essence, nutrition cream, massage cream, pack, gel, makeup base, foundation, powder, lipstick, patch, eye cream, eye essence, cleansing cream, cleansing foam, cleansing water, a cleanser, hair shampoo, hair conditioning, hair treatment, hair essence, hair lotion, scalp hair tonic, scalp essence, hair gel, hair spray, hair pack, body lotion, body cream, body oil and body essence.

### Method for obtaining the peptide composition

Conventional methods to produce cGP includes their synthesis from amino acids followed by cyclization. These conventional methods are typically complicated because of the need to remove the organic solvent and resin used for solid-phase synthesis.

For example, Hayasaka et al. (Food Science and Technology Research. 2016. 22(4):477-483) describes a method to obtain cGP peptide from natural collagen, involving the steps:
i) enzymatic hydrolysis of collagen;
ii) isolating a collagen tripeptide (CTP) fraction from the hydrolysate (by reverse-phase HPLC);
iii) heating the CTP fraction to induce cyclization to cyclic peptides including cGP peptide

The method of Hayasaka et al. enriches the amount of cGP (e.g. to >20%), and relies on first isolating the CTP fraction. Isolating the CTP is considered to remove most of the dipeptides and free amino acids, which typically contribute to the biological effect of collagen peptide compositions. Moreover, the isolation step involves increased costs and time required for production, i.e. reduced efficiency.

The present inventors discovered a method for production of cGP starting of a natural material, which has higher practical value and may therefore be a more promising alternative. In particular, it was found that heat-treating an intermediate collagen hydrolysate product with appropriate and relative amount of peptides leads to a peptide composition enriched in cGP and further comprising desirable amounts of (cyclic) di- and tripeptides. Without being bound by theory, the present inventors consider that the heating promotes cyclization of linear peptides in the intermediate collagen hydrolysate product, in particular gly-pro-x tripeptides, mostly gly-pro-hyp and gly-pro-ala peptides present in the intermediate collagen hydrolysate product. It was furthermore found that the heating to induce cyclisation considerably increases the amount of hydroxylysine and hydroxyproline. Therefore, the peptide composition of the invention may also have a characteristic content in hydroxylysine and/or hydroxyproline.

In various embodiments, the method of the invention allows obtaining the peptide composition disclosed herein.

In embodiments, the method of the invention comprises
a) providing a collagen hydrolysate, comprising at least 1.0x10⁴ ppm -1.0x10⁵ ppm glycineproline-hydroxyproline tripeptide and 5x10³- 5x10⁴ ppm glycine-proline-alanine, calculated on total weight of peptides in the collagen hydrolysate, an having a weight-average molecular weight of at least 500 Da and at most 3000 Da; and
b) heating the collagen hydrolysate, preferably at a temperature of at least 65 °C, thereby allowing to obtain a peptide composition enriched in cyclic(glycine-proline).

The collagen hydrolysate in the first step has a weight-average molecular weight of at least 500 Da and at most 3000 Da.

The collagen hydrolysate provided in step a) comprises 1.0x10⁴ - 1.0x10⁵ ppm glycine-proline-hydroxyproline tripeptide. The collagen hydrolysate (provided in step a) comprises 5.0x10³ - 5.0x10⁴ ppm, preferably 1.0x10⁴ - 3.0x10⁴ ppm glycine-proline-alanine tripeptide.

The collagen hydrolysate (provided in step a) preferably comprises glycine-proline-hydroxyproline tripeptide in an amount of 10000-100000 ppm, preferably 20000-80000 ppm. For example, the amount of glycine-proline-hydroxyproline tripeptide may be 30000-60000 ppm.

In a preferred embodiment, the collagen hydrolysate (provided in step a) comprises an amount of glycine-proline-alanine tripeptide of 5000-75000 ppm, preferably 10000-50000 ppm, more preferably 20000-40000 ppm, calculated on total weight of peptides in the collagen hydrolysate.

The collagen hydrolysate (provided in step a) preferably has an amount of collagen dipeptides of less than 5.0x10³ ppm, preferably less than 1.0x10³ ppm, more preferably less than 5.0x10² ppm, even more preferably less than 1.0x10² ppm, for example 10 - 1000 ppm or 50-500 ppm.

The collagen hydrolysate (provided in step a) preferably has an amount of free amino acids of less than 1.0x10⁵ ppm, preferably less than 5.0x10⁴ ppm, more preferably less than 1.0x10⁴ ppm, even more preferably less than 0.5x10⁴ ppm, most preferably less than 1.0x10³ ppm.

The collagen hydrolysate (provided in step a) preferably is characterized by a weight average molecular weight of at least 800 Da, more preferably at least 1000 Da. The collagen hydrolysate (provided in step a) preferably is characterized by a weight average molecular weight of at most 2500 Da, more preferably at most 2000 Da. In embodiments, the collagen hydrolysate (provided in step a) preferably has a weight-average molecular weight (Mw) in the range of 500 Da - 3000 Da, preferably 750 Da - 3000 Da, more preferably 1000 - 2500 Da, even more preferably 1200 - 2000 Da. The collagen hydrolysate may have a weight average molecular weight of at least 500 Da, 600 Da, 700 Da, 800 Da, 900 Da, 1000 Da, 1100 Da, 1200 Da, 1300 Da, 1400 Da, 1500 Da, 1750 Da, 2000 Da, 2250 Da, 2500 Da, 2750 Da or 3000 Da. In addition or alternatively, the collagen hydrolysate may have a weight average molecular weight of at most 3000 Da, or 2500 Da, or 2250 Da, or 2000 Da or 1750 Da, or 1700 Da, or 1650 Da or 1600 Da.

In preferred embodiments, the collagen hydrolysate has a molecular weight distribution defined by a polydispersity index of 1.0 - 2.0 preferably 1.2- 1.8, more preferably 1.4-1.7, even more preferably 1.5-1.6. In preferred embodiments, the collagen hydrolysate has a molecular weight distribution defined by a polydispersity of 1.1- 1.7, more preferably 1.2-1.6, even more preferably 1.3-1.5.

In embodiments of the invention, the collagen hydrolysate comprises 0 -2%, more preferably 0.2-1% by weight of peptides with a molecular weight of 0-250 Da or at most 250 Da.

In embodiments of the invention, the collagen hydrolysate comprises 0 - 2%, more preferably 0.2-1% by weight of peptides with a molecular weight of 250-450 Da.

In embodiments of the invention, the collagen hydrolysate comprises 30-50%, more preferably 35-45% by weight of peptides with a molecular weight of 450-1000 Da. In embodiments of the invention, the collagen hydrolysate comprises 0.1-10%, preferably 0.5-5%, more preferably 1-4% by weight of peptides with a molecular weight of at most 500 Da.

In embodiments of the invention, the collagen hydrolysate comprise 25-50%, more preferably 30-45% by weight of peptides with a molecular weight of 1000-2000 Da.

In embodiments of the invention, the collagen hydrolysate comprises 0.5-30%, more preferably 15-20 by weight of peptides with a molecular weight of 2000-5000 Da. In embodiments of the invention, the collagen hydrolysate comprises 0.2-5%, more preferably 0.5-2% by weight of peptides with a molecular weight of 5000-10000 Da. In embodiments of the invention, the collagen hydrolysate comprises at most 2%, preferably 0.01-2%, more preferably 0.1-1% by weight of peptides with a molecular weight more than 10000 Da, calculated on total weight of total peptides in the peptide composition.

In various embodiments, the collagen hydrolysate (provided in step a) of the method and/or the peptide composition obtainable in step b) comprises one or more cyclic (i.e. cyclo) peptides selected from the group consisting of Cyclo(Gly-Ala), Cyclo(Gly-Leu), Cyclo(Gly-Glu), Cyclo(Gly-Ser), Cyclo(Gly-Phe), Cyclo(Gly-Val), Cyclo (Leu-Hyp) and Cyclo (Ala-Hyp). In various embodiments, the collagen hydrolysate (provided in step a) of the method comprises cGP in an amount of 500-20000 ppm, preferably 1000-10000 ppm. The amount of cGP in the collagen hydrolysate may be preferably 1000-5000 ppm, more preferably 2000-4000 ppm.

In various embodiments, the collagen hydrolysate (provided in step a) of the method comprises hydroxylysine in an amount of 1-200 ppm, preferably 2-100 ppm. The amount of hydroxylysine in the collagen hydrolysate may be preferably 10-50 ppm, more preferably 15-30 ppm. In various embodiments, the collagen hydrolysate (provided in step a) of the method comprises hydroxyproline in an amount of 100-2500 ppm, preferably 200-1500 ppm. The amount of hydroxyproline in the collagen hydrolysate may be preferably 300-1000 ppm, more preferably 400-800 ppm.

In various embodiments, the collagen hydrolysate (provided step a) of the method and/or the peptide composition obtainable in step b) comprises one or more linear tripeptides selected from the group consisting of Gly-Pro-Hyp, Gly-Pro-Pro, Gly-Pro-Ala, Gly-Ala-Hyp, Gly-Ala-Pro, Gly-Leu- Hyp, Gly-Glu-Hyp, Gly-Glu-Arg, Gly-Pro-Arg, Gly-Ser-Hyp, Gly-Ala-Lys, Gly-Ala-Arg, Gly-Pro-Lys, Gly-Pro-Ser, Gly-Phe-Hyp, Gly-Pro-Gln, Gly-Ala-Ala, Gly-Pro-Val, Gly-Asp-Ala, Gly-Glu-Ala, Gly-Ala-Asp, Gly-Arg-Hyp and Ala-Hyp-Gly.

In various embodiments, the collagen hydrolysate (provided in step a) of the method and/or the peptide composition obtainable in step b) comprises one or more linear dipeptides selected from the group consisting of Gly-Pro, Pro-Gly, Gly-Hyp, Pro-Hyp, Hyp-Gly, Ala-Hyp and Pro-Ala.

The heat treatment (in step b) may cause a dehydration and/or cyclization reaction. The heating time and/or temperature is variable, and can be adapted according to the yield of peptides that is desired. In addition or alternatively, the heating time can be dependent on the hydrolysis step(s) performed. For instance, the present inventors found that the hydrolysis time and/or heat treatment time may be reduced when consecutively hydrolysing with two or more enzymes. The heat treatment may be performed at a temperature of preferably 70 °C or higher, such as 70 - 120 °C, preferably 80 - 110 °C, more preferably 90 - 100 °C. The temperature is preferably the temperature of the liquid formulation wherein the collagen hydrolysate is provided.

The heat treatment in the context of the current invention is preferably for 8 - 36 hours, more preferably 12 - 24 hours, even more preferably 16-20 hours. In various embodiments, the heating time is at least 0.5, 1, 2, 3, 4, 5, 6, 8, 10, 12, 14, 16, 18 or 20 h, and in addition or alternatively at most 48, 36, 30, 24, 20, 18, 16, 14, 12, 10, 8, 6, 5 or 4 h.

### Method for obtaining the collagen hydrolysate

The present inventors found that the digestion of collagen with a microbial collagenase having cleavage activity at Xaa-|-Gly bonds obtains an intermediate collagen hydrolysate product rich in glycine-proline-x tripeptide sequences (e.g. at least 1.0x10⁴ ppm, preferably 2.0x10⁴ - 2.0x10⁵ ppm gly-pro-x tripeptides) - in particular glycine-proline-hydroxyproline (e.g. 1.0x10⁴ - 1.0x10⁵ ppm) and glycine-proline-alanine tripeptide (e.g. 5x10³ - 5x10⁴ ppm) sequences - and rich in further collagen di- and tripeptides. In addition, the intermediate collagen hydrolysate is characterized by a relatively low amount of dipeptides and free amino acids and further desirable molecular weight distribution.

The "x" amino acid in the "gly-pro-x" tripeptide in the context of the current invention can be any amino acid residue (arginine, lysine, histidine, phenylalanine, tyrosine, leucine, isoleucine, methionine, valine, alanine, glycine, proline, glutamic acid, glutamine, serine, threonine, aspartic acid, asparagine, tryptophan, cysteine, hydroxyproline, or hydroxylysine).

The terms "dipeptide" and "tripeptide" in the context of the current invention refer to respectively linear dipeptide and linear tripeptide (i.e. different from cyclic peptides) unless stated otherwise.

In embodiments, the method pertains to a method for obtaining a collager hydrolysate, preferably a collagen hydrolysate as disclosed herein.

The method for obtaining the collagen hydrolysate preferably involves hydrolysing a collagen and/or gelatin starting material, more preferably with a microbial collagenase.

In an embodiment, the collagen and/or gelatin starting material is preferably comprised of or derived from one or more (animal) collagen-containing tissues.

The term "collagen" in the context of the current invention means an amino acid sequence comprising a repeating (Gly-X-Y) sequence, preferably comprising at least 2, 3, 4, 5, 10, 20, 50, 100, 200, 300, or 400 sequences containing the sequence Gly-X-Y, where X and Y are an amino acid residue independently chosen from each other, but X and/or Y are more preferably proline. The "collagen" preferably has a sequence found in native collagen in one or more animal species. In addition, or alternatively, the "collagen" can mean a full-length sequence or fragment or subunit thereof of (native) collagen, preferably one or more of collagen types I - XXVII, more preferably one or even more of type I, II, III, IV, V, or X collagen, even more preferably one or more of type I, II, III or IV collagen, or any combination thereof. For example, the term "collagen" may refer to an alpha-1(I), alpha-2(I), alpha-1(II) or alpha-1(III) chain, or a fragment thereof. The term "collagen" encompasses the triple helix structure as formed by three subunits as present in native collagen. The gelatin in the context of the current invention may be derived from partial hydrolysis of one or more types of collagen selected from collagen types I - XXVII, preferably one or more of type I, II,III or IV collagen, or any combination thereof. In a preferred embodiments of the invention, the collagen is derived from one or more of skin, cartilage, bone, scale, sternum, or the collagen may be a mixture of collagens derived from different tissues. The term "skin" as disclosed herein encompasses "hide", i.e. meaning the outer covering of large animals such as from bovine (species) or any other large animals. The terms "skin" and "hide" may herein be used interchangeably, and may refer the outer coverage of an animal, irrespective of size.

As used herein, the term "collagen hydrolysate" means a mix of short chains of amino acids derived from a collagen-containing material having native (full-length) collagen, preferably obtained via hydrolysis steps, including enzymatic hydrolysis (also called enzymatic hydrolyzation). The "collagen hydrolysate" herein may encompasses collagen which is subjected to hydrolysis or partial hydrolysis. The term "collagen hydrolysate" may be used interchangeably and synonymously with the terms "collagen hydrolysate" "hydrolysed collagen" or "collagen peptide". The term "gelatin" in the context of the current invention means a mixture of water-soluble peptides and/or proteins derived from partial hydrolysis of collagen. The gelatin of the invention may be type A gelatin, type B gelatin or a combination thereof. The hydrolysis may be performed by any acid or alkali condition, or by enzymatic hydrolysis, as known in the art. Depending on the physical and chemical methods of the partial hydrolysis, the molecular weight of the peptides can fall within a broad range (e.g. 10-95 kDa). The partial hydrolysis provides the gelatin the ability to hold water and its gelling capacity, typically distinguishing it from "collagen hydrolysate", i.e. products obtained by further hydrolysis of collagen.

In embodiments, the "collagen hydrolysate" disclosed herein is produced from a collagen-containing material in a one-step process. In embodiments, the "collagen hydrolysate" disclosed herein is produced via the intermediate gelatin stage, in which e.g. type A and/or type B gelatin (e.g. a mixture of type A and type B gelatin) are preferably used.

In embodiments, the collagen may be derived from partial hydrolysis of one or more types of collagen and/or of one more animals or species of animals, such as collagen of bovine (species), pig (species), chicken and fish (species), or combinations thereof.

For hydrolysis, the collagen-containing material (e.g. collagen, gelatin) is preferably provided in a liquid formulation together with one or more enzymes.

The hydrolysis of the collagen and/or gelatin in the context of the current invention preferably involves enzymatic hydrolysis. Enzymes suitable for enzymatic hydrolysis in the context of the current invention include one or more selected from the group consisting of collagenase such as a collagenase, acid protease, amino protease, serine protease, alkaline protease, neutral protease, flavour protease, complex protease, thiol protease, bromelain, metalloprotease, aspartame, protease, carboxypeptidase, pepsin, chymotrypsin, trypsin, cathepsin K, chymotrypsin, papain and subtilisin.

In a preferred embodiment, the enzyme cleaves at Xaa-|-Gly bonds. In an embodiment, the enzyme cleaves at Y-Gly bonds in repeating Gly-X-Y triplets in a collagen sequence, wherein X or Y represent any amino acid residue. In an embodiment, the enzyme cleaves at -Gly bonds in the sequence -Xaa-Gly-Pro- or -Pro-Xaa-Gly-Pro-. In an embodiment, the enzyme is a collagenase, more preferably a microbial collagenase.

It is preferred if the context of the present invention that the enzyme does not produce free amino acids or only produces relatively low amounts of free amino acids.

The present inventors found that the digestion of collagen with a microbial collagenase having cleavage activity at Xaa-|-Gly bonds obtains an intermediate collagen hydrolysate product rich in glycine-proline-x tripeptide sequences- in particular rich in glycine-proline hydroxyproline and glycine-proline-alanine tripeptide sequences - and comprising further collagen di- and tripeptides.

In an embodiment, the enzyme used for hydrolysis is preferably not ginger powder and/or an enzyme cleaving preferentially at peptide bonds with Pro/Hyp at the P2 position. Without being bound by theory, the present inventors consider that cleavage of collagen peptide bonds with Pro/Hyp at the P2 position leads to a collagen hydrolysate which contains substantial amounts of X-Hyp-Gly-type peptides, and which upon heating do not achieve the desirable relative amounts of cyclic (peptides). Without being bound by theory, the present inventors consider that cleaving peptide bonds with Pro/Hyp at the P2 position does not lead to sufficient enrichment of glycine-proline-x tripeptide sequences - in particular glycine-proline hydroxyproline and glycine-proline-alanine tripeptide sequences - in the intermediate collagen hydrolysate product.

In the notation "Xaa-|-Gly", "Xaa" represents any amino acid residue, "|" signifies the cleavage site, "Gly" specifically indicates glycine. An enzyme with cleavage activity at Xaa-|-Gly bonds will cleave peptide bonds where the amino acid on the N-terminal side can be any amino acid ("Xaa"), and the amino acid on the C-terminal side is glycine.

In an embodiment, the microbial collagenase in the context of the current invention more preferentially cleaves at Xaa-|-Gly bonds when compared to other bonds in collagen.

In an embodiment, the microbial collagenase in the context of the current invention more preferentially cleaves at Xaa-|-Gly bonds when compared to the cleavage activity at peptide bonds with Pro/Hyp at the P2 position.

In the context of the current invention, the cleavage activity of a collagenase can be determined by subjecting the collagen to the collagenase, mapping the peptides generated by enzymatic digestion (e.g. by mass spectrometry analysis), and determine the relative abundance of each peptide to quantify the extent of cleavage at different sites. This method is suitable to assess the substrate specificity of an enzyme.

The microbial collagenase in the context of the invention is preferably isolated from *Bacillus cereus, Clostridium histolyticum, Empedobacter collagenolyticum, Pseudomonas marinoglutinosa, Streptomyces sp.* such as *Streptomyces violaceoruber, Vibrio sp.,* such as *Vibrio B-30* (ATCC 21250) or *Vibrio alginolyticus* (previously *Achromobacter iophagus*) or Clostridium histolyticum. In an embodiment, the microbial collagenase is from *Streptomyces* sp. In an embodiment, the microbial collagenase is produced by a self-cloning-strain, wherein the strain is preferably a strain as disclosed herein.

In an embodiment, the microbial collagenase belongs to peptidase family M9. In an embodiment, the microbial collagenase is classified according to Enzyme Commission (EC) number as EC 3.4.24 (accepted name "Metallo endopeptidases"), preferably EC 3.4.24.3 (accepted name "microbial collagenase").

In a preferred embodiment, the method comprises hydrolysing gelatin with a first enzyme to obtain gelatin hydrolysate and hydrolysing the gelatin hydrolysate with a second enzyme to obtain collagen hydrolysate.

In a preferred embodiment, the method for obtaining the collagen hydrolysate comprises the steps:
- obtaining a gelatin by alkali or acid hydrolysis of collagen,
- hydrolysing the gelatin with a first enzyme to obtain a gelatin hydrolysate, wherein the first enzyme is preferably selected from the group consisting of collagenase, acid protease, amino protease, serine protease, alkaline protease, neutral protease, flavour protease, complex protease, thiol protease, bromelain, metalloprotease, aspartame, protease, carboxypeptidase, pepsin, chymotrypsin, trypsin, cathepsin K, chymotrypsin, papain and subtilisin and/or wherein the first enzyme is preferably not a microbial collagenase having cleavage activity at Xaa-|-Gly bonds,
- hydrolysing the gelatin hydrolysate with a second enzyme which is preferably a microbial collagenase having cleavage activity at Xaa-|-Gly bonds in collagen, to obtain a collagen hydrolysate.

The present inventors found that consecutive hydrolysis with a first enzyme (preferably not cleaving at Xaa-|-Gly bonds) followed by a second enzyme (preferably cleaving at Xaa-|-Gly bonds) can reduce the hydrolysis time and/or time needed for cyclization of peptides including cGP. In addition or alternatively, consecutive hydrolysis with a first enzyme (preferably not cleaving at Xaa-|-Gly bonds) followed by a second enzyme (preferably cleaving at Xaa-|-Gly bonds) may improve the obtained peptide composition and its biological activity (e.g. antioxidant, anti-neuro(inflammatory) effect and/or anti-fibrotic effect.

For hydrolysis, the material is preferably subjected to an enzyme concentration of 100-10000 ppm, preferably 500-5000 ppm, more preferably 1000-3000 ppm, even more preferably 1500 - 2500 ppm, calculated on the total weight of the collagen-containing material and the liquid formulation. In a preferred embodiment, the hydrolysis involves subjecting the collagen and/or gelatin to a microbial collagenase having cleavage activity at Xaa-|-Gly bonds at a concentration of 500-5000 ppm, preferably 1000-3000 ppm, more preferably 1500 - 2500 ppm, calculated on the total weight of the collagen-containing material and the liquid formulation.

In embodiments, the hydrolysis is performed at pH 6.5-9.5, preferably pH 7.0-9.0, more preferably pH 7.5-8.5. In embodiments of the invention, the hydrolysis is performed at 15-45 °C, preferably 20-40 °C, more preferably 25-35°C. In embodiments of the invention, the hydrolysis is performed for 4-24 h, preferably 8 - 20 h, more preferably 12 - 16 h.

### General definitions

The terms 'comprising' or 'to comprise' and their conjugations are used in the context of the current invention in their non-limiting sense to indicate that items following the word are included, but items not specifically mentioned are not excluded.

Reference to an element by the indefinite article 'a' or 'an' does not exclude the possibility that more than one of the elements is present, unless the context clearly requires that there be one and only one of the elements. The indefinite article 'a' or 'an' thus usually means 'at least one'.

In the context of the current invention, a level is considered "increased" or "decreased" when it is at least 1% (such as at least 5%, 10%, 15%, 20%, 25%, 30%, 35%, 40%, 45%, or 50%) higher or lower, respectively, than the corresponding level in a control or reference. In addition or alternatively, a level is considered increased or decreased when it is statistically significantly higher or lower, respectively, compared to a level in a control or reference (including compared to an earlier time point), irrespective of the size of the change. The term "to reduce" may in the context of the current invention be used interchangeably with the term "to decrease".

The terms "administer" or "administration" in the context of the current invention mean providing a compound, substance, or composition to a subject consuming it. A subject consuming a compound, substance, or composition may administer it to himself/herself. In such a case the term "administer" can be read as "take in".

The term "subject" in the context of the current invention means any animal (e.g. a mammal), preferably a human. The term "subject" can be used interchangeably with "person" or "individual" in the context of the current invention. The "subject" can be a patient.

The term "preventing" means to ensure that a subject will not develop a condition. An intervention is herein also considered to be a form of "preventing" when a condition is delayed, reduced in severity and/or reduced in incidence, even when the condition is not entirely kept from happening. The terms "preventing" or "prevention" can be used in a therapeutic and/or a non-therapeutic effect. The "preventing", or "prevention" can be defined by any delay, change in severity, and/or change in incidence, such as of at least 5, 10, 20, 30, 40, 50, 60, 70, 80, 90, 100%, as compared to a control or reference as measured by any standard technique.

In the context of the current invention, "treating" means that an intervention reduces and/or cures a condition once the condition is already existing. The terms "treating" or "treatment" can be used in a therapeutic and/or a non-therapeutic effect. The treatment can for example be any reduction in severity, incidence, and/or frequency of the condition, such as of at least 5%, 10%, 20%, 40%, 50%, 60%, 80%, 90%, 95%, or completely (100%), as compared to a control or reference as measured by any standard technique.

The value "0" in the context of the present invention can also encompass "essentially zero" or "essentially free of", meaning that the amount is not measurable according to a standard technique in the field and/or is below a certain threshold e.g. rendering the amount negligible , preferably below 0.1, more preferably below 0.01, even more preferably below 0.001, most preferably below 0.0001, for instance when referring to %-values.

### FIGURE LEGENDS

**Figure 1****.** Radical scavenging activity for cGP, gly-pro-hyp, gly-pro-ala, AP (additive effect and actual effect) and ascorbic acid (positive control). The additive effect of the combination cGP+gly-pro-hyp is compared to the actual (synergistic) effect of the combination in AP. The radical scavenging activities for cGP, gly-pro-hyp or gly-pro-ala were normalized to their respective concentrations present in the peptide composition (concentrations according to Table 3).
**Figure 2****.** Effect of AP on hydrogel contraction in a fibroblast contraction assay. Fibroblast contraction after 14 days of exposure to different concentrations of AP or TGF-β1 (positive control to stimulate fibrosis). Each symbol represents an independent experiment and donor, n=3. At least 100 cells per hydrogel were counted. Repeated Measures One-way ANOVA with Dunnett multiple comparison test. *p<0,05; **p<0,01; ***p<0,005; ****p<0,001
**Figure 3****.** Effect of AP on hydrogel contraction in an adipose stromal cell contraction assay. Adipose stromal cell contraction after 14 days of exposure to different concentrations of AP or TGF-β1 (positive control to stimulate fibrosis). Each symbol represents an independent experiment and donor, n=3. At least 100 cells per hydrogel were counted. Repeated Measures One-way ANOVA with Dunnett multiple comparison test. *p<0,05; **p<0,01; ***p<0,005; ****p<0,001
**Figure 4****.** Effect of AP on differentiation of fibroblasts into myofibroblasts. Myofibroblast differentiation in fibroblasts after 14 days of exposure to different concentrations of AP or TGF-β1 (positive control to stimulate fibrosis). Each symbol represents an independent experiment and donor, n=3. At least 100 cells per hydrogel were counted. Repeated Measures One-way ANOVA with Dunnett multiple comparison test. *p<0,05; **p<0,01; ***p<0,005; ****p<0,001
**Figure 5****.** Effect of AP on differentiation of adipose stromal cells into myofibroblasts. Myofibroblast differentiation in adipose stromal cells after 14 days of exposure to different concentrations of AP or TGF-β1 (positive control to stimulate fibrosis). Each symbol represents an independent experiment and donor, n=3. At least 100 cells per hydrogel were counted. Repeated Measures One-way ANOVA with Dunnett multiple comparison test. *p<0,05; **p<0,01; ***p<0,005; ****p<0,001
**Figure 6****.** Effect of treatment on spontaneous alternation behavior in D-Galactose treated mice. With * indicating a significant difference (p<0.05) in comparison to the 60% baseline (dotted line) which is to be expected behavior of the animals in this model.
**Figure 7****.** Effect of AP on the learning profile of D-Galactose treated mice in the Morris water maze test with p<0.05 (NaCl/H₂O vs. D-Gal/H₂O), p<0.05 [D-Gal/AP (1722 mg/kg) vs. D-Gal/H₂O].
**Figure 8****.** Interleukin-6 (IL-6) concentration (mean ± SEM) in the brain cortex (left), brain hippocampus (middle) and plasma (right). With *significant difference with negative control group ("*NaCl*/*H₂O*") and ^{#}significant difference with positive control group ("*D-Gal*/*H₂O*").
**Figure 9****.** Tumor necrosis factor alpha (TNF-α) concentration (mean ± SEM) in the brain cortex (left), hippocampus (middle) and plasma (right). With *significant difference with negative control group ("*NaCl*/*H₂O*") and ^{#}significant difference with positive control group ("D-*Gal*/*H₂O"*).
**Figure 10****.** Brain-derived neurotrophic factor (BDNF) concentration (mean ± SEM) in brain cortex (left), hippocampus (middle) and plasma (right). With *significant difference with negative control group ("*NaCl*/*H₂O*") and ^{#}significant difference with positive control group ("D-*Gal*/*H₂O*").
**Figure 11****.** Interferon gamma (INF-γ) concentration (mean ± SEM) in brain cortex (left) and hippocampus (right). With *significant difference with negative control group (*"NaCl*/*H₂O"*) and ^{#}significant difference with positive control group (*"D-Gal*/*H₂O"*).
**Figure 12****.** TGF-β concentration in the brain cortex (mean ± SEM). With *significant difference with negative control group ("*NaCl*/*H₂O*") and ^{#}significant difference with positive control group ("*D-Gal*/*H₂O*").
**Figure 13****.** Monocyte chemoattractant protein-1 (MCP-1) concentration in the plasma (mean ± SEM). With *significant difference with negative control group ("*NaCl*/*H₂O*") and ^{#}significant difference with positive control group ("*D-Gal*/*H₂O*").
**Figure 14****.** Neurofilament light polypeptide (NfL) concentration (mean ± SEM) in the brain cortex (left) and hippocampus (right With *significant difference with negative control group ("*NaCl*/*H₂O*") and ^{#}significant difference with positive control group ("*D-Gal*/*H₂O*").

### EXAMPLES

### Example 1: preparation method and characteristics of a cGP-enriched peptide composition

### Enzymatic hydrolysis

Porcine gelatin (20% w/w) was enzymatically hydrolysed (pH 8, T=30 °C, t=16 h) with an enzyme having cleavage activity at Xaa-|-Gly bonds in collagen (EC 3.4.24.3, from *Streptomyces* sp.). The enzyme was used at a concentration of 2000 ppm.

Comparative tests (not shown) indicated that this specific enzyme activity led to a favourable molecular weight distribution and peptide composition as starting point for further cyclisation.

Peptides were determined by UPLC-MS analysis (ultra-performance liquid chromatography-mass spectrometry).,

The collagen hydrolysate obtained by the enzymatic hydrolysis is also referred to as the "precursor".

Table 1 shows the peptide composition of the precursor. The peptide composition was determined for two separate replicates produced according to the same protocol.

**Table 1. Peptide composition of the precursor. Values are in parts per million (ppm).**

| | **Replicate #1** | **Replicate #2** | **Average** |
|---|---|---|---|
| | **(ppm)** | **(ppm)** | **(ppm)** |
| **cGP** | 3226 | 2338 | 2782 |
| **Hydroxylysine** | 22 | 20 | 21 |
| **Hydroxyproline** | 670 | 503 | 587 |
| **Gly-pro-ala** | 20247 | 22978 | 21613 |
| **Gly-pro-hyp** | 39427 | 46169 | 42798 |

Table 2 shows the molecular weight characteristics of the precursor. The peptide composition was determined for two separate replicates produced according to the same protocol.

**Table 2. Molecular weight characteristic of the precursor (average values for two replicates). Mn=number average molecular weight, Mw=weight average molecular weight.**

| | | |
|---|---|---|
| **Mn** | | 1000-1100 Da |
| **Mw** | | 1500-1600 Da |
| **Polydispersity** | | 1.4 |
| **Distribution** | | |
| | • 0-500 Da | 2% |
| | • 500-1000 Da | 38% |
| | • 1-2 kDa | 41% |
| | • 2-5 kDa | 17% |
| | • 5-10 kDa | 1% |
| | • >10kDa | <1% |

### Cyclisation by heating

The formation of cGP by cyclization was further induced by heating at 90°C for 24 h.

The peptide composition obtained after heating and cyclization is herein also referred to as "AP".

Table 3 shows the peptide composition of AP. The peptide composition was determined for three separate replicates which were produced according to the same protocol.

**Table 3. Peptide composition of AP. Values are in parts per million (ppm).**

| | **Replicate #1** | **Replicate #2** | **Replicate #3** | **Average** |
|---|---|---|---|---|
| | **(ppm)** | **(ppm)** | **(ppm)** | **(ppm)** |
| **cGP** | 45944 | 67684 | 47203 | 53611 |
| **Hydroxylysine** | 610 | 901 | 697 | 736 |
| **Hydroxyproline** | 13811 | 19041 | 14241 | 15698 |
| **Gly-pro-ala** | 194 | 217 | 150 | 187 |
| **Gly-pro-hyp** | 5047 | 5972 | 4318 | 5112 |
| **Pro-gly-pro** | 2 | <4 | <1 | 2 |
| **Pro-hyp-gly** | 1 | 1 | 1 | 1 |
| **Pro-hyp** | 11 | 13 | 6 | 10 |
| **Hyp-gly** | 18 | 22 | 17 | 19 |
| **Pro-gly** | 831 | 986 | 679 | 832 |
| **Gly-pro** | 2791 | 3858 | 2408 | 3019 |
| **Ala-pro** | 32 | 44 | 32 | 36 |

Table 4 shows the molecular weight characteristics of AP. The peptide composition was determined for three separate replicates which were produced according to the same protocol.

**Table 4. Molecular weight characteristics of AP. Mn=number average molecular weight, Mw=weight average molecular weight.**

| | | |
|---|---|---|
| **Mn** | | 950-1050 Da |
| **Mw** | | 1500-1600 Da |
| **Polydispersity** | | 1.6 |
| **Distribution** | | |
| | • 0-500 Da | 11% |
| | • 500-1000 Da | 24% |
| | • 1-2 kDa | 43% |
| | • 2-5 kDa | 20% |
| | • 5-10 kDa | 2% |
| | • >10kDa | <1% |

**Table 5** shows a comparison of the molecular weight characteristics of the precursor (before heating and cyclisation) and AP (after heating and cyclisation), based on the average values for the individual replicates measured.

**Table 5. Comparison of the molecular weight characteristic of the precursor (average values for two replicates) and AP (average values of three replicates). Mn=number average molecular weight, Mw=weight average molecular weight.**

| | | **Precursor (before cyclisation)** | **AP (after cyclisation)** |
|---|---|---|---|
| **Mn** | | 1000-1100 Da | 950-1050 Da |
| **Mw** | | 1500-1600 Da | 1500-1600 Da |
| **Polydispersity** | | 1.4 | 1.6 |
| **Distribution** | | | |
| | • 0-500 Da | 2% | 11% |
| | • 500-1000 Da | 38% | 24% |
| | • 1-2 kDa | 41% | 43% |
| | • 2-5 kDa | 17% | 20% |
| | • 5-10 kDa | 1% | 2% |
| | • >10kDa | <1% | <1% |

When comparing the molecular weight data, a similar overall Mn and Mw is observed before and after cyclisation. However, a change in molecular distribution can be seen when comparing the product before and after cyclisation.

As can be seen in **Table 5,** before cyclisation ~2% of peptides are below 500 Da while after cyclisation this increases to ~11%, whereas before cyclisation ~38% of peptides are in the 500-1000 Da range while after cyclisation this decreases to ~24%.

Since there appears to be shift in certain relatively lower molecular weight fractions due to cyclisation, a further closer characterization was performed of the molecular weight distribution for the 0-250 Da, 250-450 Da and 450-1000 Da fractions **(Table 6).**

**Table 6. Comparison of the molecular weight characteristics of the precursor (average values for two replicates) and AP (average values of three replicates). MW =molecular weight**

| | | **Precursor (before cyclisation)** | **AP (after cyclisation)** |
|---|---|---|---|
| **MW Distribution** | | | |
| | • 0-250 Da | <1% | <1% |
| | • 250-450 Da | <1% | 10% |
| | • 450-1000 Da | 39% | 25% |

As can be seen in **Table 6,** before cyclisation <1% of peptides have a molecular weight in the range 250-450 Da while after cyclisation this increases to ~10%, whereas before cyclisation ~39% of peptides are in the 450-1000 Da range while after cyclisation this decreases to ~25%. Thus, cyclisation appears to shift peptides from the 450-1000 Da range to the 250-450 Da range and leads to a product with a characteristic peak in the 250-450 Da molecular weight range. It is considered that cGP (Mw=154 Da) contributes to this characteristic peak because cGP, due to its shape, shows a typical peak in a size exclusion chromatogram at 250-450 Da.

**Table 7** shows a comparison of the peptide composition of the precursor (before heating and cyclisation) and AP (after heating and cyclisation), based on the average values for the individual replicates measured.

**Table 7. Comparison of peptide composition of the precursor (average values for two replicates) and AP (average values for three replicates). Values are in parts per million (ppm).**

| | **Precursor (before cyclisation)** | **AP (after cyclisation)** | **Fold change** |
|---|---|---|---|
| **cGP** | 2782 | 53611 | ~20x increase |
| **Hydroxylysine** | 21 | 736 | ~35x increase |
| **Hydroxyproline** | 587 | 15698 | ~27x increase |
| **Gly-pro-ala** | 21613 | 187 | ~116x decrease |
| **Gly-pro-hyp** | 42798 | 5112 | ~8x decrease |

It can be seen that the amount of cGP increases considerably (~x20) after cyclisation.

The amounts of hydroxylysine (~x35) and hydroxyproline (~x27) also increase considerably after cyclisation. Looking at the tripeptide concentrations, the amounts of Gly-pro-ala and Gly-pro-hyp drop dramatically after cyclisation, which can be explained by the formation of cGP. Thus, the comparison of the peptides before and after cyclisation shows that cGP was efficiently generated from Gly-pro-x peptide sequence, in particular from Gly-pro-ala or Gly-pro-hyp.

### Example 2: radical scavenging activity of the cGP enriched peptide composition

Example 2 shows the radical scavenging activity of AP. The AP was produced as described in Example 1.

### Methods

### Materials and reagents

- 2,2-Diphenyl-1-picrylhydrazyl (DPPH) (R092) (394.32 g/mol)
- Methanol
- Distilled water

### DPPH assay protocol

The assay was performed in a covered 96-well plate (end-volume per well was 200 µL) according to following protocol:
i) Prepare a stock solution of DPPH in methanol
   - Final concentration per well is 75 µM
ii) Prepare a stock solution of the test hydrolysates in water
   - Dilution series is prepared to obtain a final concentration per well of 1 mg/mL
iii) Final solvent concentration per well is 50/50 methanol/water (v/v)
iv) Add DPPH stock solution to each test well in triplicate
   - Include positive controls (DPPH without test substance, in triplicate)
v) Add hydrolysate to each test well in triplicate
   - Include negative controls (test substance without DPPH, in triplicate)
vi) Mix well and incubate mixture in the dark for 30 min at room temperature
vii) Measure absorbance at 517 nm of the different conditions
viii) Normalize the measured absorbance for the test conditions (sample + DPPH) based on the absorbance of the negative controls (test substance without DPPH)
ix) Calculate the mean (± standard deviation) absorbance (n=3) for each test sample and for the DPPH positive controls (without test substance)
x) The percentage reduction in absorption (compared to the DPPH positive control) is given by the following formula: 1 - (mean absorbance test sample/mean absorbance DPPH positive control)

### Results

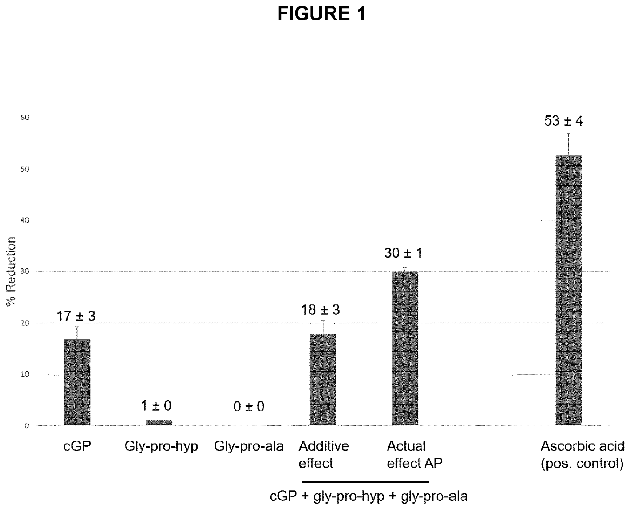

**Figure 1** shows the radical scavenging activity for cGP, gly-pro-hyp, gly-pro-ala, AP (additive effect and actual effect) and ascorbic acid (positive control). The radical scavenging activities for cGP, gly-pro-hyp, or gly-pro-ala were normalized to their respective concentrations present in AP (concentrations according to Table 3). The additive effect is the theoretical additive effect which is the sum of the normalized radical scavenging activities for cGP, gly-pro-hyp and gly-pro-ala.

It was found that the cGP present in AP accounts for 17% of the reduction in DPPH, the gly-pro-hyp present in AP accounts for 1% of the reduction in DPPH, the gly-pro-ala present in AP accounts for 0% of the reduction in DPPH.

The additive effect of cGP, gly-pro-hyp and gly-pro-ala is therefore an 18% reduction in DPPH.

However, the combination of cGP, gly-pro-hyp and gly-pro-ala as present in AP actually leads to ~30% reduction in DPPH. Thus, cGP, gly-pro-hyp and gly-pro-ala have a synergistic effect in reducing DPPH.

It was furthermore found that further dipeptides (e.g. Pro-Hyp, Gly-pro) and tripeptides in AP can further improve the radical scavenging activity of AP.

### Example 3: effects of AP on fibrosis

Example 3 shows the effects of AP in different models of fibrosis.

### Methods

### Materials

The AP was produced as described in Example 1.

### Fibroblast and Adipose stromal cell (ASC) isolation

Normal skin (including subcutaneous fat) was obtained from patients undergoing abdominal dermolipectomy or breast reduction surgery and collected anonymously. Fibroblasts and adipose stromal cells (ASCs) were isolated from skin and subcutaneous fat tissue respectively. Fat was removed from the dermis and epidermis, and cut into pieces. The epidermis was removed from the dermis after dispase incubation. Both dermis and adipose tissue were incubated separately in collagenase type II (Gibco, Invitrogen, Paisly, UK)/dispase II (Roche, Mannheim, Germany) solution for 2 h at 37 °C. Cells were passed through a 40 µm cell strainer and cultured in a 37 °C, 5% CO₂ atmosphere in DMEM supplemented with 1% penicillin/streptomycin (Invitrogen, Carlsbad, CA, USA) and 1% UltroserG (BioSepra SA, Cergy-Saint-Christophe, France). Cells from n=3 donors were used for experiments at passage 2-4.

### Cell culture assay

Collagen 1 was isolated from rat tails and reconstituted in 0.1% acetic acid (4 mg/ml). Fibroblasts were seeded in 4 mg/ml collagen I solution (cell concentration: 2x10⁵ cells/ml) and 1 ml hydrogel/well was poured into 12 wells plates. Hydrogels were allowed to polymerize for 2 hours at 37 °C. Hydrogels were detached from the well surface to allow contraction and culture medium was added to the wells. Medium with peptides was filtered using a 0.2 µm filter and allowed to cool to 37 °C before adding to the cultures. Transforming growth factor-β1 (TGF-β1) was used as a control to stimulate fibrosis. Three times per week the culture medium (including peptides) was changed. Each experimental condition had an intra-experimental replicate. After two weeks of culture, the hydrogels were fixed in 4% paraformaldehyde and embedded in paraffin.

### Myofibroblast staining

The presence of myofibroblasts was determined by immunohistochemical staining for alpha Smooth Muscle Actin (α-SMA). Hydrogels were fixed in 4% paraformaldehyde and embedded in paraffin. 5 µm sections were dehydrated, incubated 1 hour with mouse-anti-human α-SMA (1:200, clone 1A4, M0851, DAKO) followed a blocking step with Brightvision Gold for 15 minutes and BrightVision Poly-HRP-Anti Mouse/Rabbit IgG Ruby for 30 minutes. 3-Amino-9-ethylcarbazole was used as a substrate and slides were counterstained with hematoxylin. The percentage of α-SMA positive cells was determined by manually counting at least 50 cells per hydrogel (counted on average 100 per hydrogel).

### Results

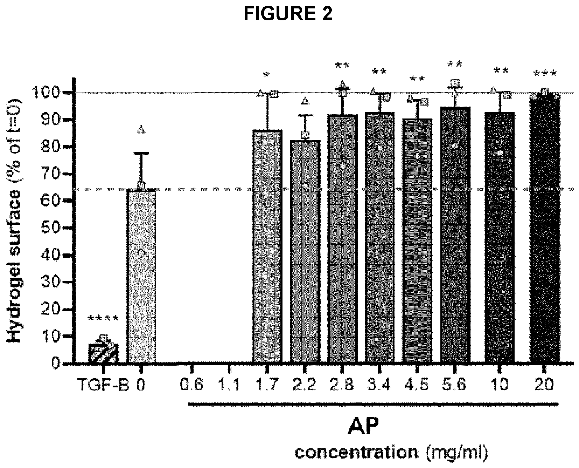

**Figure 2** shows that AP reduces hydrogel contraction in the fibroblast contraction assay. The maximum effect of AP on fibroblasts was observed at the lowest tested concentration of 1.7 mg/ml.

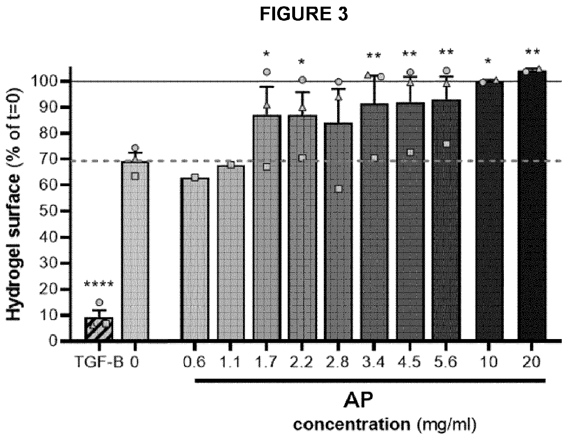

**Figure** 3 shows that AP reduces hydrogel contraction in the adipose stromal cell (ASC) contraction assay. The maximum effect of AP on ASCs was observed at 1.7 mg/ml. There was a trend towards increased activity of AP over pure cGP at low concentrations AP (i.e. 0.15-0.25 mg/ml).

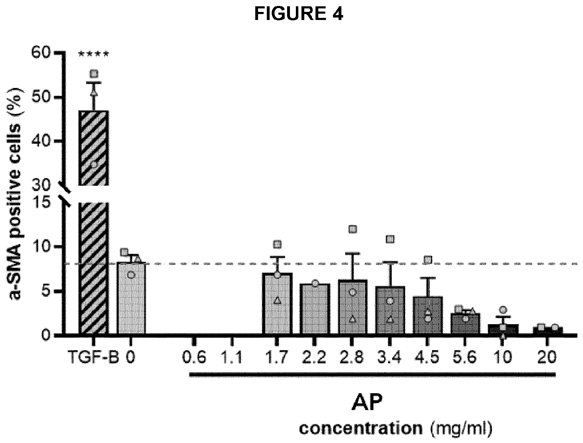

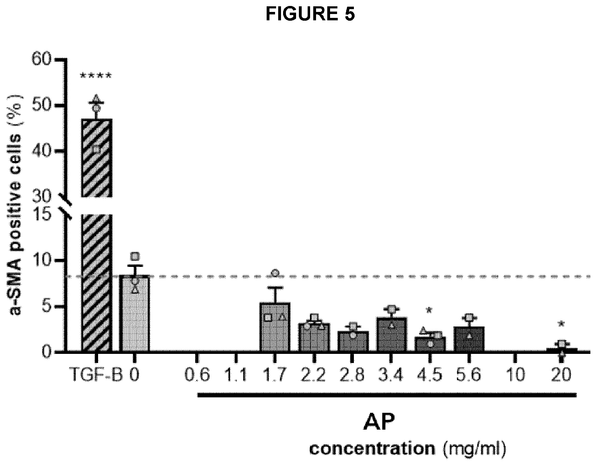

At the end of the 14 days culture period, hydrogels were processed for paraffin embedding and the percentage of α-SMA positive cells determined by immunohistochemistry. In accordance with the contraction data, the percentage of α-SMA positive cells was significantly increased in TGF-β1 stimulated hydrogels compared to unexposed control. A clear trend in reduced percentage of α-SMA positive cells was observed with increasing concentrations of AP, both for fibroblasts **(****Figure 4****)** and ASCs **(****Figure 5****).**

**Figure 4** shows that AP reduces differentiation of fibroblasts into myofibroblasts in a dose-dependent manner, as indicated by the reduced percentage of α-SMA positive cells.

**Figure 5** shows that AP reduces differentiation of adipose stromal cells into myofibroblasts in a dose-dependent manner, as indicated by the reduced percentage of α-SMA positive cells.

It was furthermore found that AP ameliorates the overproduction of collagen I in connective tissue cells such as fibroblasts. AP also appears to ameliorate the production of collagen III and V in connective tissue cells such as fibroblasts.

It was found that AP can inhibit macrophage enhanced contraction of fibroblast populated hydrogels, suggesting anti-inflammatory properties. It was also found that AP stimulates monocyte derived dendritic cell (moDC) maturation and stimulate macrophage skewing towards the inflammatory M1 phenotype rather than the fibrotic M2 phenotype.

Overall, without being bound by theory, blocking or reducing the overproduction of collagen(s) and/or modulating the immune response by AP may be mechanism of actions that (further) lead to the prevention of fibrosis.

The results show that AP is surprisingly effective in the prevention of fibrosis, at least in part by preventing the contraction and/or myofibroblast differentiation in connective tissue cells. There is a dose-dependent effect of cyclic glycine-proline in preventing fibrosis.

### Example 4:

Example 4 shows the different neuro-protective properties of AP in a D-galactose mouse model of ageing. The AP was produced as described in Example 1.

### Methods

### Study setup

Sixty (60) male Swiss mice (3 months old at receipt) were used for the experiment. An overview of the test compounds and treatment groups are shown in **Table 8.** The test compounds were administered by oral route once per day, 7 days per week during 69 days. For induction of ageing, D-Galactose (D-GAL) was administered subcutaneously (SC) once daily during 54 days (i.e. from 15 days after initiating treatment and onwards). Thus, treatment started 2 weeks prior to induction of brain ageing. D-Gal was administered one hour after the oral administration of the test compounds. Between D43 - D54, three different behavioral tests were used to monitor the effects of the test compounds:
i) the spontaneous alternation procedure in the Y-maze (assessing spatial working memory) on D43;
ii) the spatial learning in the Morris water maze (MWM) test from D46 to D50, and
iii) the passive avoidance response (assessing contextual long-term memory) with training on D53 and retention session on D54.

The day after the behavioral test (on D55) animals were sacrificed for sample collection (plasma, brain cortex and brain hippocampus) for determination of the following biochemical parameters:
- Inflammatory markers: tumor necrosis factor alpha (TNF-α), interleukin-6 (IL-6)
- Cognitive markers: brain-derived neurotrophic factor (BDNF)
- Complementary biomarkers of aging/neurodegeneration: interferon gamma (IFN-γ), transforming growth factor-beta (TGF-β), neurofilament light polypeptide (NfL), monocyte chemoattractant protein-1 (MCP-1)

Frozen cortex and hippocampus samples were cut into small parts, homogenized in sterile phosphate-buffered saline (PBS) completed with protease inhibitors at 5 mg tissue per 100 µl of PBS and sonicated 3 times during 10 seconds on ice. Then, the homogenate was centrifuged for 30 minutes at 10,000 rpm at 4°C, and the supernatant was analyzed immediately by ELISA. For all analyzed samples, cortex and hippocampus supernatants were diluted at 1/5 in sterile PBS enriched with a protease inhibitor cocktail. All dilutions were performed at 4 °C to avoid protein degradation. Standard curve biomarker dilution was performed at specific concentrations. IL-6, TNF-α, BDNF, INF-y, TGF-β and NfL quantification was performed in duplicate for each sampled using 100 µl of sample per well in 96 well plates by the ELISA method.

**Table 8. Overview of treatment groups and designations investigated in the mouse D-galactose model.**

| **Group and designation** | **# animals** | **Treatment** | **Induction of brain ageing** |
|---|---|---|---|
| Group 1: Negative control | 12 | Vehicle (H₂O) | Saline (NaCl) |
| "NaCl/H₂O" | | D0-D69 | D15-D69 |
| Group 2: Positive control | 12 | Vehicle (H₂O) | D-Gal 150 mg/kg |
| *"D-Gal*/*H₂O"* | | D0-D69 | D15-D69 |
| Group 3: AP Dose 1 | 12 | AP 172 mg/kg | D-Gal 150 mg/kg |
| *"D-Gal*/*H₂O (172 mg*/*kg)"* | | D0-D69 | D15-D69 |
| Group 4: AP Dose 2 | 12 | AP1722 mg/kg | D-Gal 150 mg/kg |
| *"D-Gal*/*H₂O (172 mg*/*kg)"* | | D0-D69 | D15-D69 |

### Results

### General health monitoring and behavioral patterns

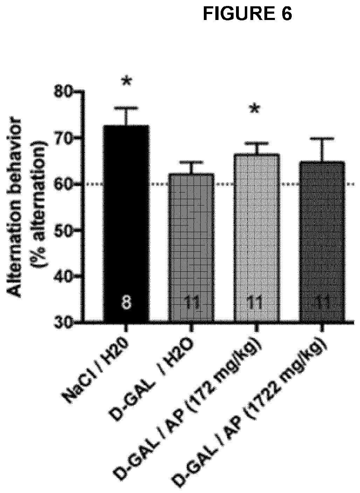

No adverse effects on weight gain were observed in any of the treatment groups, during the course of the study. A loss in spontaneous alternation based on the Y-maze test was observed in the positive control group ("*D-Gal*/*H₂O"*). Treatment with the lowest dose of AP induced a significant improvement on alternation behavior. The other treatments had a similar trend compared to the baseline of 60% **(****Figure 6****).** The negative control group (*"NaCl*/*H₂O*") displayed a percent of spontaneous alternation significantly higher than 60% (p=0.0153), which was considered characteristic of behavior of mice in the Y-maze. The positive control group produced a loss of spontaneous alternation (p=0.0301).

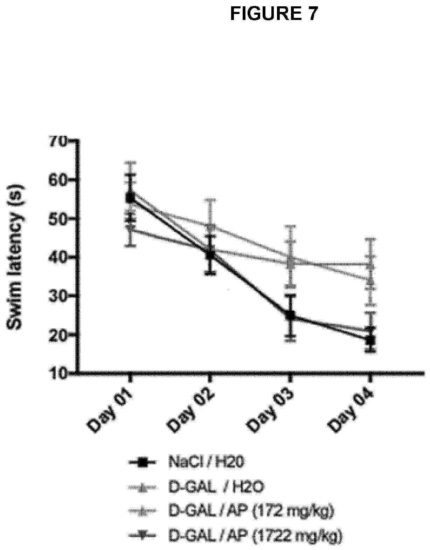

The MWM test was conducted as a tool to study spatial memory and learning **(****Figure 7****).** Control animals were able to learn the location of the hidden platform as shown by a decrease of the time of swim needed to find it at the occasion of daily training from day 1 to day 4. The positive control group ("*D-Gal*/*H₂O"*) displayed a significant deficit in the learning of the position of the hidden platform compared to the negative control group ("*NaCl*/*H₂O"*) as given by a significantly higher swim latency (p=0.0439). Treatment of D-GAL injected animals with AP produced an improvement of learning. For the AP 1722 mg/kg group (p=0.0164) the curve is almost similar to that of the negative control group ("*NaCl*/*H₂O"*)*.*

### Influence on brain biochemistry parameters in the D-galactose mouse model

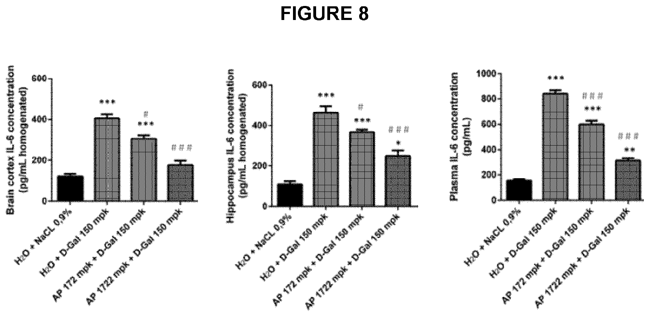

A significant increase of IL-6 concentration was observed in the brain cortex, hippocampus and plasma of the positive control animals ("*D-Gal*/*H₂O"*) compared to the negative control animals (*"NaCl*/*H₂O"*), all with p≤0.001 **(****Figure 8****).** The animals treated with AP 172 mg/kg showed a significant decrease of IL-6 concentration in the brain cortex and plasma compared to the positive control group. A strong and significant decrease of IL-6 concentration was observed in the brain, hippocampus and plasma of animals treated with AP 1722 mg/kg compared to the positive control group. This overall suggests a positive effect of this compound for this biomarker.

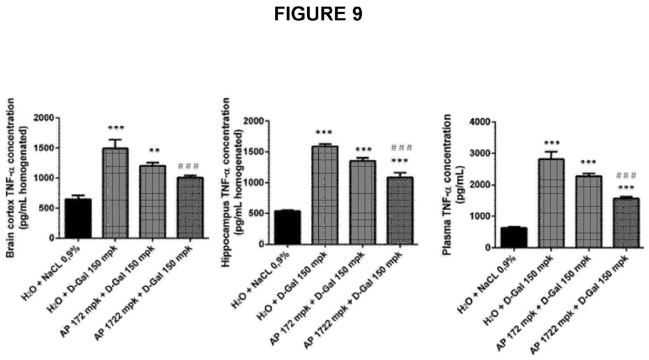

Treatment with D-GAL also induced a significant increase of TNF-α concentration in the brain cortex, hippocampus and plasma of the positive control group ("*D-Gal*/*H₂O"*) compared to the negative control animals ("*NaCl*/*H₂O"*), all with p≤0.001 **(****Figure 9****).** A decrease of TNF-α concentration was observed in the brain cortex, hippocampus and plasma of animals treated with AP at 172 mg/kg and AP at 1722 mg/kg, compared to the animals from the positive control group. This overall suggests a positive effect of AP for the TNF-α biomarker.

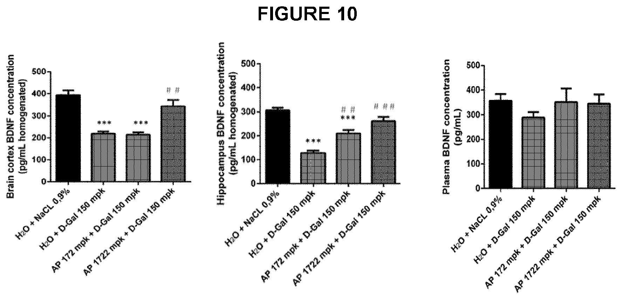

Furthermore, a significant decrease of BDNF concentration was observed in the brain cortex and hippocampus of the positive control group ("*D-Gal*/*H₂O"*) compared to the negative control animals ("*NaCl*/*H₂O"*) with p≤0.001 **(****Figure 10****).** The same trend was observed for the BDNF concentration in plasma. A significant increase of BDNF concentration was observed in the hippocampus of animals treated with AP172 mg/kg compared to the positive control group. Significant increases of BDNF concentration were observed in the brain and hippocampus of animals treated with AP at 1722 mg/kg compared to the positive control group.

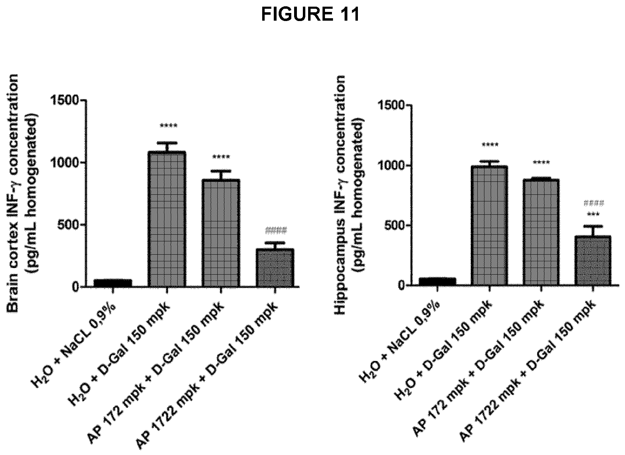

Moreover, a significant increase of INF-γ concentration was observed in the brain cortex and hippocampus of the positive control group ("*D-Gal*/*H₂O"*) compared to the negative control animals ("*NaCl*/*H₂O"*) with p≤0.001 **(****Figure 11****).** The animals treated with AP 172 mg/kg or AP at 1722 mg/kg presented a decrease of INF-γ concentration in the brain in this D-Gal mouse model.

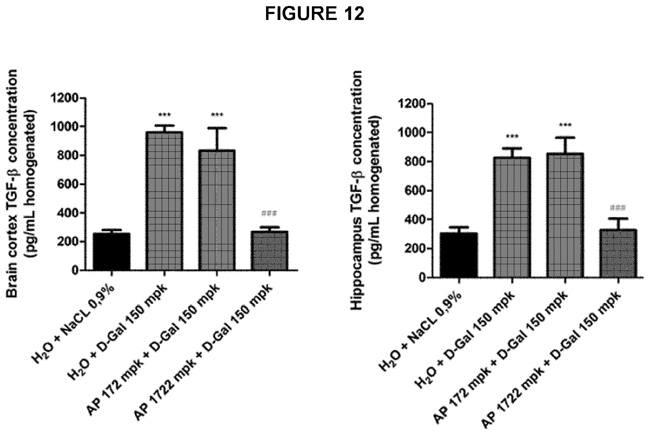

A significant increase of TGF-β concentration was observed in the brain cortex and hippocampus of the positive control animals ("*D-Gal*/*H₂O"*) compared to the negative control animals (group 1) with p≤0.001 **(****Figure 12****).** A particularly strong and significant decrease of TGF-β concentration was observed in the brain cortex and hippocampus of animals treated with AP 1722 mg/kg.

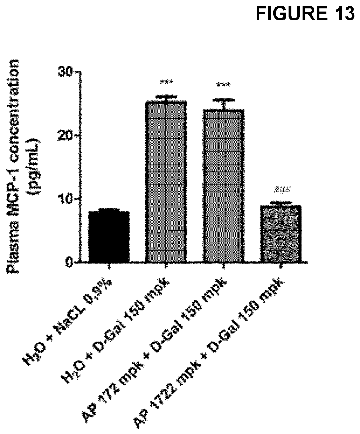

The MCP-1 marker was measured on plasma samples for the different groups. A significant increase of MCP-1 concentration was observed in the plasma of the positive control group ("*D-Gal*/*H₂O"*) in comparison with the negative control animals (*"NaCl*/*H₂O"*)*.* A particularly strong and significant decrease of MCP-1 concentration was observed in the plasma of animals treated with AP 1722 mg/kg **(****Figure 13****).**

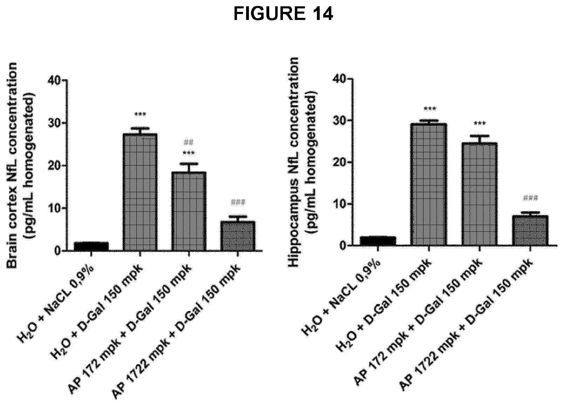

Lastly, NfL was measured in the brain cortex, hippocampus and plasma of the animals. A significant increase of NfL concentration was observed in brain cortex and hippocampus of the positive control animals ("*D-Gal*/*H₂O"*) compared to the negative control animals ("*NaCl*/*H₂O"*). No significant increase of plasma NfL concentration was observed in the positive control animals compared to the negative control animals. The animals treated with AP 172 mg/kg presented a decrease of NfL concentration in the brain cortex compared to the positive control group. In comparison with the positive control group, a particularly strong decrease of NfL was observed in the brain cortex and hippocampus of animals treated with AP 1722 mg/kg **(****Figure 14****).**

Based on the present results, it is considered that the administration of AP may be particularly effective in improving cognitive function, e.g. ability to focus, attention span, learning ability or ameliorating neurodevelopmental conditions such as ADHD.

## Claims

1. Peptide composition, comprising
1.0x10⁴ ppm or more cyclic(glycine-proline);
1.0x10³ ppm or more glycine-proline-hydroxyproline tripeptide; and
1.0x10¹ ppm or more glycine-proline-alanine tripeptide,
wherein the ppm is calculated on total weight of peptides in the peptide composition.

2. Peptide composition according to claim 1, comprising 1.0x10⁴ - 2.0x10⁵ ppm cyclic(glycine-proline).

3. Peptide composition according to claim 1 or 2, comprising 1.0x10³ - 1.0x10⁴ ppm glycine-proline-hydroxyproline tripeptide.

4. Peptide composition according to any one of claims 1-3, comprising 1.0x10¹ - 1.0x10³ ppm glycine-proline-alanine tripeptide.

5. Peptide composition according to any one of the previous claims, wherein the peptide composition has a weight-average molecular weight of 1000 - 2000 Da, preferably 1200-1800 Da.

6. Peptide composition according to any one of the previous claims, the composition comprising
- 0-2% by weight of peptides with a molecular weight of at most 250 Da; and
- 5-15% by weight of peptides with a molecular weight of 250-450 Da; and
- 15-35 % by weight of peptides with a molecular weight of 450-1000 Da, calculated on total weight of peptides in the peptide composition; and/or
- 35-50% by weight of peptides with a molecular weight of 1000-2000 Da; and
- 15-30% by weight of peptides with a molecular weight of 2000-5000 Da; and
- 0.5-5% by weight of peptides with a molecular weight of 5000-10000 Da; and
- at most 2%, preferably 0.01-1%, by weight of peptides with a molecular weight more than 10000 Da, calculated on total weight of peptides in the peptide composition.

7. Peptide composition according to any one of claims 1-6, for use as a medicament.

8. Peptide composition according to any one of claims 1-6, for use in preventing and/or treating a condition selected from the group consisting of a neurological condition and fibrosis.

9. Peptide composition for use according to claim 8, wherein the neurological condition is one or more selected from the group consisting of central nervous system inflammation, central nervous system inflammation injury, movements conditions or disorders, neuromotor disease, neuromuscular disease, neurosensory disease, psychotic conditions or disorders, depressive conditions or disorders, anxiety conditions or disorders, nervous system and/or brain damage, cognitive conditions or disorders, personality conditions or disorders, mood conditions or disorders, migraine, epilepsy or convulsive disorders, neurodegenerative condition or disorder, autistic disorders, neuropathy, a neurodevelopmental disorder and neurotoxicity.

10. Peptide composition for use according to claim 8, wherein the fibrosis is one or more selected from the group consisting of medical implant-related fibrosis, surgery-related fibrosis, organ-specific fibrosis, post-surgical adhesion formation, skin fibrosis, cellulite, Dupuytren's contracture, a hypertrophic scar, a keloid, liver fibrosis, pulmonary fibrosis, pancreatic fibrosis, kidney fibrosis, vascular fibrosis, endometriosis, atrial fibrosis and heart fibrosis,
or wherein the fibrosis is a symptom in a condition selected from the group consisting of fatty liver disease, chronic obstructive pulmonary disease, asthma and systemic sclerosis.

11. Peptide composition for use according to any one of claims 7-10, wherein the peptide composition is administered orally and/or at a total daily dose of 0.1-20 g.

12. Use of the composition according to any one of claims 1-6 in non-therapeutic improvement of cognitive function, wherein the cognitive function is preferably selected from the group consisting of learning ability, memory, spatial memory, problem solving capacity, capacity for remembering, attention span, concentration, ability of language processing, spatial ability, speech, language skills, ability to focus, mental alertness and reaction time.

13. Method for obtaining a peptide composition comprising cyclic(glycine-proline) according to any one of claims 1-6, the method comprising a) providing a collagen hydrolysate comprising 1.0x10⁴ - 1.0x10⁵ ppm glycine-proline-hydroxyproline tripeptide and 5x10³ - 5x10⁴ ppm glycine-proline-alanine, calculated on total weight of peptides in the collagen hydrolysate, and having a weight-average molecular weight of at least 500 Da and at most 3000 Da; and b) heat-treating the collagen hydrolysate at a temperature of at least 65 °C.

14. Method according to claim 13, wherein the collagen hydrolysate provided in step a) has a weight-average molecular weight of at least 800 Da and at most 2500 Da, preferably at least 1000 Da and at most 2000 Da, even more preferably at least 1200 Da and at most 1800 Da.

15. Method according to claim 13 or 14, wherein the collagen hydrolysate provided in step a) has:
- 0-2% by weight of peptides with a molecular weight of at most 250 Da; and
- 0-2% by weight of peptides with a molecular weight of 250-450 Da; and
- 30-50 % by weight of peptides with a molecular weight of 450-1000 Da; and/or
- 25-50% % by weight of peptides with a molecular weight of 1000-2000 Da; and
- 0.5-30% by weight of peptides with a molecular weight of 2000-5000 Da; and
- 0.2-5% by weight of peptides with a molecular weight of 5000-10000 Da; and
- at most 2%, preferably 0.01-1%, by weight of peptides with a molecular weight more than 10000 Da, calculated on total weight of peptides in the peptide composition.

## Patentansprüche

1. Peptidzusammensetzung, umfassend
1,0x10⁴ ppm oder mehr cyclisches (Glycin-Prolin),
1,0x10³ ppm oder mehr Glycin-Prolin-Hydroxyprolin-Tripeptid und
1,0x10¹ ppm oder mehr Glycin-Prolin-Alanin-Tripeptid,
wobei der ppm-Wert auf das Gesamtgewicht von Peptiden in der Peptidzusammensetzung berechnet wird.

2. Peptidzusammensetzung nach Anspruch 1, umfassend 1,0x10⁴-2,0x10⁵ ppm cyclisches (Glycin-Prolin).

3. Peptidzusammensetzung nach Anspruch 1 oder 2, umfassend 1,0x10³- 1,0x10⁴ ppm Glycin-Prolin-Hydroxyprolin-Tripeptid.

4. Peptidzusammensetzung nach einem der Ansprüche 1-3, umfassend 1,0x10¹ - 1,0x10³ ppm Glycin-Prolin-Alanin-Tripeptid.

5. Peptidzusammensetzung nach einem der vorhergehenden Ansprüche, wobei die Peptidzusammensetzung ein gewichtsmittleres Molekulargewicht von 1000 - 2000 Da, vorzugsweise 1200-1800 Da, aufweist.

6. Peptidzusammensetzung nach einem der vorhergehenden Ansprüche, wobei die Zusammensetzung Folgendes umfasst:
- 0-2 Gew.-% Peptide mit einem Molekulargewicht von höchstens 250 Da und
- 5-15 Gew.-% Peptide mit einem Molekulargewicht von 250-450 Da und
- 15-35 Gew.-% Peptide mit einem Molekulargewicht von 450-1000 Da, berechnet auf das Gesamtgewicht von Peptiden in der Peptidzusammensetzung; und/oder
- 35-50 Gew.-% Peptide mit einem Molekulargewicht von 1000-2000 Da und
- 15-30 Gew.-% Peptide mit einem Molekulargewicht von 2000-5000 Da und
- 0,5-5 Gew.-% Peptide mit einem Molekulargewicht von 5000-10.000 Da und
- höchstens 2 Gew.-%, vorzugsweise 0,01-1 Gew.-%, Peptide mit einem Molekulargewicht von mehr als 10.000 Da, berechnet auf das Gesamtgewichts von Peptiden in der Peptidzusammensetzung.

7. Peptidzusammensetzung nach einem der Ansprüche 1-6 zur Verwendung als Medikament.

8. Peptidzusammensetzung nach einem der Ansprüche 1-6 zur Verwendung bei der Prävention und/oder Behandlung eines Leidens, der aus der Gruppe bestehend aus einem neurologischen Leiden und Fibrose ausgewählt ist.

9. Peptidzusammensetzung zur Verwendung nach Anspruch 8, wobei es sich bei dem neurologischen Leiden um eines oder mehrere aus der Gruppe bestehend aus Zentralnervensystementzündung, Zentralnervensystementzündungsverletzung, Bewegungsleiden oder -störungen, neuromotorischer Erkrankung, neuromuskulärer Erkrankung, neurosensorischer Erkrankung, psychotischen Leiden oder Störungen, depressiven Leiden oder Störungen, Angstleiden oder -störungen, Nervensystem- und/oder Gehirnschädigung, kognitiven Leiden oder Störungen, Persönlichkeitsleiden oder - störungen, affektiven Leiden oder Störungen, Migräne, Epilepsie oder konvulsiven Störungen, neurodegenerativem Leiden oder neurodegenerativer Störung, autistischen Störungen, Neuropathie, einer neurologischen Entwicklungsstörung und Neurotoxizität handelt.

10. Peptidzusammensetzung zur Verwendung nach Anspruch 8, wobei es sich bei der Fibrose um eine oder mehrere aus der Gruppe bestehend aus durch medizinische Implantate bedingter Fibrose, operationsbedingter Fibrose, organspezifischer Fibrose, Bildung von postoperativen Adhäsionen, Hautfibrose, Cellulite, Dupuytren-Kontraktur, eine hypertrophische Narbe, ein Keloid, Leberfibrose, Lungenfibrose, Bauchspeicheldrüsenfibrose, Nierenfibrose, Gefäßfibrose, Endometriose, Vorhoffibrose und Herzfibrose handelt oder wobei es sich bei der Fibrose um ein Symptom bei einem Leiden ist, das aus der Gruppe bestehend aus Fettlebererkrankung, chronisch-obstruktiver Lungenerkrankung, Asthma und systemischer Sklerose ausgewählt ist.

11. Peptidzusammensetzung zur Verwendung nach einem der Ansprüche 7-10, wobei die Peptidzusammensetzung oral und/oder in einer täglichen Gesamtdosis von 0,1-20 g verabreicht wird.

12. Verwendung der Zusammensetzung nach einem der Ansprüche 1-6 bei der nichttherapeutischen Verbesserung der kognitiven Funktion, wobei die kognitive Funktion vorzugsweise aus der Gruppe bestehend aus Lernfähigkeit, Gedächtnis, räumlichem Gedächtnis, Problemlösungsfähigkeit, Erinnerungsfähigkeit, Aufmerksamkeitsspanne, Konzentration, Sprachverarbeitungsfähigkeit, räumlichem Vorstellungsvermögen, Sprache, Sprachfähigkeiten, Fokussierungsfähigkeit, geistiger Wachsamkeit und Reaktionszeit ausgewählt ist.

13. Verfahren zum Erhalten einer Peptidzusammensetzung, die cyclisches (Glycin-Prolin) umfasst, nach einem der Ansprüche 1-6, wobei das Verfahren Folgendes umfasst:
a) Bereitstellen eines Kollagenhydrolysats, das 1,0x10⁴- 1,0x10⁵ ppm Glycin-Prolin-Hydroxyprolin-Tripeptid und 5x10³ - 5x10⁴ ppm Glycin-Prolin-Alanin, berechnet auf das Gesamtgewicht von Peptiden in dem Kollagenhydrolysat, umfasst und ein gewichtsmittleres Molekulargewicht von mindestens 500 Da und höchstens 3000 Da aufweist; und
b) Wärmebehandeln des Kollagenhydrolysats bei einer Temperatur von mindestens 65 °C.

14. Verfahren nach Anspruch 13, wobei das in Schritt a) bereitgestellte Kollagenhydrolysat
ein gewichtsmittleres Molekulargewicht von mindestens 800 Da und höchstens 2500 Da, vorzugsweise mindestens 1000 Da und höchstens 2000 Da, noch weiter bevorzugt mindestens 1200 Da und höchstens 1800 Da, aufweist.

15. Verfahren nach Anspruch 13 oder 14, wobei das in Schritt a) bereitgestellte Kollagenhydrolysat Folgendes aufweist:
- 0-2 Gew.-% Peptide mit einem Molekulargewicht von höchstens 250 Da und
- 0-2 Gew.-% Peptide mit einem Molekulargewicht von 250-450 Da und
- 30-50 Gew.-% Peptide mit einem Molekulargewicht von 450-1000 Da und/oder
- 25-50 Gew.-% Peptide mit einem Molekulargewicht von 1000-2000 Da und
- 0,5-30 Gew.-% Peptide mit einem Molekulargewicht von 2000-5000 Da und
- 0,2-5 Gew.-% Peptide mit einem Molekulargewicht von 5000-10.000 Da und
- höchstens 2 Gew.-%, vorzugsweise 0,01-1 Gew.-%, Peptide mit einem Molekulargewicht von mehr als 10.000 Da, berechnet auf das Gesamtgewichts von Peptiden in der Peptidzusammensetzung.

## Revendications

1. Composition de peptides, comprenant
1,0x10⁴ ppm ou plus de (glycine-proline) cyclique ;
1,0x10³ ppm ou plus de tripeptide glycine-proline-hydroxyproline ; et
1,0x10¹ ppm ou plus de tripeptide glycine-proline-alanine,
dans laquelle le ppm est calculé sur le poids total de peptides dans la composition de peptides.

2. Composition de peptides selon la revendication 1, comprenant 1,0x10⁴ à 2,0x10⁵ ppm de (glycine-proline) cyclique.

3. Composition de peptides selon la revendication 1 ou 2, comprenant 1,0x10³ à 1,0x10⁴ ppm de tripeptide glycine-proline-hydroxyproline.

4. Composition de peptides selon l'une quelconque des revendications 1 à 3, comprenant 1,0x10¹ à 1,0x10³ ppm de tripeptide glycine-proline-alanine.

5. Composition de peptides selon l'une quelconque des revendications précédentes, dans laquelle la composition de peptides a un poids moléculaire moyen en poids de 1 000 à 2 000 Da, de préférence de 1 200-1 800 Da.

6. Composition de peptides selon l'une quelconque des revendications précédentes, la composition comprenant
- de 0 à 2 % en poids de peptides d'un poids moléculaire maximal de 250 Da; et
- 5 à 15 % en poids de peptides ayant un poids moléculaire de 250 à 450 Da ; et
- 15 à 35 % en poids de peptides ayant un poids moléculaire de 450 à 1 000 Da, calculé sur le poids total de peptides dans la composition de peptides ; et/ou
- 35 à 50 % en poids de peptides ayant un poids moléculaire de 1000 à 2000 Da ; et
- 15 à 30 % en poids de peptides ayant un poids moléculaire de 2000 à 5000 Da ; et
- 0,5 à 5 % en poids de peptides ayant un poids moléculaire de 5000 à 10000 Da ; et
- au plus 2 %, de préférence 0,01 à 1 % en poids de peptides ayant un poids moléculaire supérieur à 10 000 Da, calculé sur le poids total de peptides dans la composition de peptides.

7. Composition de peptides selon l'une quelconque des revendications 1 à 6, à être utilisée comme médicament.

8. Composition de peptides selon l'une quelconque des revendications 1 à 6, destinée à être utilisée dans la prévention et/ou le traitement d'un état choisi dans le groupe constitué par un état neurologique et une fibrose.

9. Composition de peptides destinée à être utilisée selon la revendication 8, dans laquelle l'état neurologique est un ou plusieurs choisis dans le groupe constitué par une inflammation du système nerveux central, une lésion inflammatoire du système nerveux central, des troubles ou affections du mouvement, une maladie neuromotrice, une maladie neuromusculaire, une maladie neurosensorielle, des troubles ou affections psychotiques, des troubles ou affections dépressifs, des troubles ou affections anxieux, des lésions du système nerveux et/ou du cerveau, des troubles ou affections cognitifs, des troubles ou affections de la personnalité, des troubles ou affections de l'humeur, la migraine, l'épilepsie ou les troubles convulsifs, une affection ou un trouble neurodégénératif, des troubles autistiques, une neuropathie, un trouble neurodéveloppemental et une neurotoxicité.

10. Composition de peptides destinée à être utilisée selon la revendication 8, dans laquelle la fibrose est une ou plusieurs fibroses choisies dans le groupe constitué par une fibrose médicale liée à l'implant, une fibrose chirurgicale, une fibrose spécifique d'un organe, la formation d'adhérences post-opératoires, une fibrose cutanée, de la cellulite, une contracture de Dupuytren, une cicatrice hypertrophique, une chéloïde, une fibrose hépatique, une fibrose pulmonaire, une fibrose pancréatique, une fibrose rénale, une fibrose vasculaire, une endométriose, une fibrose auriculaire et une fibrose cardiaque,
ou dans laquelle la fibrose est un symptôme dans un état choisi dans le groupe constitué par une stéatose hépatique, une bronchopneumopathie chronique obstructive, l'asthme et une sclérose systémique.

11. Composition de peptides destinée à être utilisée selon l'une quelconque des revendications 7 à 10, dans laquelle la composition de peptides est administrée par voie orale et/ou à raison d'une dose quotidienne totale de 0,1-20 g.

12. Utilisation de la composition selon l'une quelconque des revendications 1-6 dans l'amélioration non thérapeutique d'une fonction cognitive, dans laquelle la fonction cognitive est de préférence choisie dans le groupe constitué par la capacité d'apprentissage, la mémoire, la mémoire spatiale, la capacité de résolution de problèmes, la capacité de mémorisation, la durée d'attention, la concentration, la capacité de traitement du langage, l'aptitude spatiale, la parole, les compétences linguistiques, la capacité de concentration, la vigilance mentale et le temps de réaction.

13. Procédé d'obtention d'une composition de peptides comprenant une (glycine-proline) cyclique selon l'une quelconque des revendications 1-6, le procédé comprenant
a) la fourniture d'un hydrolysat de collagène comprenant 1,0x10⁴ à 1,0x10⁵ ppm de tripeptide glycine-proline-hydroxyproline et 5x10³ à 5x10⁴ ppm de glycine-proline-alanine, calculé sur le poids total de peptides dans l'hydrolysat de collagène, et ayant un poids moléculaire moyen en poids d'au moins 500 Da et d'au plus 3 000 Da ; et
b) le traitement thermique de l'hydrolysat de collagène à une température d'au moins 65 °C.

14. Procédé selon la revendication 13, dans lequel l'hydrolysat de collagène fourni à l'étape
a) présente un poids moléculaire moyen en poids d'au moins 800 Da et d'au plus 2 500 Da, de préférence d'au moins 1 000 Da et d'au plus 2 000 Da, de manière encore plus préférée d'au moins 1 200 Da et d'au plus 1 800 Da.

15. Procédé selon la revendication 13 ou 14, dans lequel l'hydrolysat de collagène fourni à l'étape a) présente :
- 0 à 2 % en poids de peptides ayant un poids moléculaire d'au plus à 250 Da ; et
- 0 à 2 % en poids de peptides ayant un poids moléculaire de 250 à 450 Da ; et
- 30 à 50 % en poids de peptides ayant un poids moléculaire de 450 à 1 000 Da ; et/ou
- 25 à 50 % en poids de peptides ayant un poids moléculaire de 1 000 à 2 000 Da ; et
- 0,5 à 30 % en poids de peptides ayant un poids moléculaire de 2000 à 5000 Da ; et
- 0,2 à 5 % en poids de peptides ayant un poids moléculaire de 5000 à 10000 Da ; et
- au plus 2 %, de préférence 0,01 à 1 % en poids de peptides ayant un poids moléculaire supérieur à 10 000 Da, calculé sur le poids total de peptides dans la composition de peptides.
